(19) Europäisches Patentamt
European Patent Office
Office européen des brevets

(11) Publication number : **0 511 791 A2**

(12) # EUROPEAN PATENT APPLICATION

(21) Application number : **92303712.1**

(22) Date of filing : **24.04.92**

(51) Int. Cl.[5] : **C07D 471/04,** A61K 31/44, // (C07D471/04, 221:00, 209:00)

(30) Priority : **30.04.91 GB 9109246**

(43) Date of publication of application : **04.11.92 Bulletin 92/45**

(84) Designated Contracting States : **AT BE CH DE DK ES FR GB GR IT LI LU MC NL PT SE**

(71) Applicant : **IMPERIAL CHEMICAL INDUSTRIES PLC**
**Imperial Chemical House, Millbank**
**London SW1P 3JF (GB)**

(72) Inventor : **Edwards, Martin Paul**
**Alderley Park**
**Macclesfield, Cheshire, SK10 4TG (GB)**
Inventor : **Pearce, Robert James**
**Alderley Park**
**Macclesfield, Cheshire, SK10 4TG (GB)**
Inventor : **Masek, Brian Bernard, ICI Americas Inc.**
**Concord Pike & New Murphy Road**
**Wilmington DE (US)**

(74) Representative : **Smith, Stephen Collyer et al**
**ICI Group Patents Services Dept. PO Box 6**
**Shire Park Bessemer Road**
**Welwyn Garden City Herts, AL7 1HD (GB)**

(54) **Pyrrolopyridine derivatives as Angiotensin inhibitors.**

(57) The invention concerns pharmaceutically useful compounds of the formula I, in which $R^1$, $R^2$, $R^3$, $R^4$, $R^5$, $R^6$, X and Z have the various meanings defined herein, and their non-toxic salts, and pharmaceutical compositions containing them. The novel compounds are of value in treating conditions such as hypertension and congestive heart failure. The invention further concerns processes for the manufacture of the novel compounds and the use of the compounds in medical treatment.

EP 0 511 791 A2

This invention concerns novel nitrogen derivatives and, more particularly, novel pyrrolopyridine derivatives which possess pharmacologically useful properties in antagonising at least in part one or more of the actions of the substances known as angiotensins, and in particular of that known as angiotensin II (hereinafter referred to as "AII"). The invention also concerns pharmaceutical compositions of the novel compounds for use in treating diseases or medical conditions such as hypertension, congestive heart failure and/or hyperaldosteronism in warm-blooded animals (including man), as well as in other diseases or medical conditions in which the renin-angiotensin-aldosterone system plays a significant causative role. The invention also includes processes for the manufacture of the novel compounds and their use in treating one of the afore-mentioned diseases or medical conditions and for the production of novel pharmaceuticals for use in such medical treatments.

The angiotensins are key mediators of the renin-angiotensin-aldosterone system, which is involved in the control of homeostasis and fluid/electrolyte balance in many warm-blooded animals, including man. The angiotensin known as AII is produced by the action of angiotensin converting enzyme (ACE) on angiotensin I, itself produced by the action of the enzyme renin on the blood plasma protein angiotensinogen. AII is a potent spasmogen especially in the vasculature and is known to increase vascular resistance and blood pressure. In addition, the angiotensins are known to stimulate the release of aldosterone and hence result in vascular congestion and hypertension via sodium and fluid retention mechanisms. Hitherto there have been a number of different approaches to pharmacological intervention in the renin-angiotensin-aldosterone system for therapeutic control of blood pressure and/or fluid/electrolyte balance, including, for example, inhibiting the actions of renin or ACE. However, there remains a continuing need for an alternative approach because of the side-effects and/or idiosyncratic reactions associated with any particular therapeutic approach.

In our co-pending European Patent Application, Publication No. 399731 there is disclosed certain imidazopyridine derivatives which have AII antagonist activity.

We have now discovered that the compounds of the invention (set out below) surprisingly antagonise one or more of the actions of the substances known as angiotensins (and in particular of AII) and thus minimise the physiological effects associated with their presence in warm-blooded animals (including man) and this is the basis of the invention.

According to the invention there is provided a pyrrolopyridine derivative of the formula I (set out hereinafter, together with the other chemical formulae identified by Roman numerals) wherein $R^1$ is hydrogen, (1-8C)alkyl, (3-8C)cycloalkyl, phenyl or substituted (1-4C)alkyl, the latter containing one or more fluoro substituents or bearing a (3-8C)cycloalkyl, (1-4C)alkoxy or phenyl substituent; $R^2$ is hydrogen, (1-4C)alkyl, (1-4C)alkoxy, halogeno, trifluoromethyl, carboxy, (1-4C)alkoxycarbonyl, (3-6C)alkenyloxycarbonyl, cyano, nitro, carbamoyl, (1-4C)alkanoyl, N-alkylcarbamoyl and di-(N-alkyl)carbamoyl of up to 7 carbon atoms, amino, alkylamino and dialkylamino of up to 6 carbon atoms, 3-(1-4C)alkylureido and (1-4C)alkanoylamino; $R^3$ is selected from halogeno, (1-4C)alkoxy, hydroxy, amino, alkylamino and dialkylamino of up to 6 carbon atoms, and any of the values defined for $R^1$; $R^4$ is hydrogen, (1-4C)alkyl, (1-4C)alkyl containing one or more fluoro substituents, (1-4C)alkoxy, halogeno, carboxy, (1-4C)alkoxycarbonyl, (3-6C)alkenyloxycarbonyl, cyano, nitro, (1-4C)alkanoyl, (1-4C)alkylthio, (1-4C)alkylsulphinyl, (1-4C)alkylsulphonyl or phenylsulphonyl; $R^5$ is hydrogen, (1-8C)alkyl, (1-4C)alkyl containing one or more fluoro substituents, hydroxy(1-4C)alkyl, carboxy, (1-4C)alkoxycarbonyl, (3-6C)alkenyloxycarbonyl, cyano, (1-4C)alkanoyl, (1-4C)alkylthio, (1-4C)alkylsulphinyl, (1-4C)alkylsulphonyl, phenylsulphonyl, (3-8C)cycloalkyl, (3-8C)cycloalkyl(1-4C)alkyl, phenyl or phenyl(1-4C)alkyl; $R^6$ is selected from hydrogen, (1-4C)alkyl, (1-4C)alkoxy, halogeno, trifluoromethyl, cyano and nitro; X is phenylene optionally bearing a substituent selected from (1-4C)alkyl, (1-4C)alkoxy, halogeno, (1-4C)alkanoyl, trifluoromethyl, cyano and nitro, or X is a direct bond between the adjacent phenyl and methylene groups; Z is 1H-tetrazol-5-yl, -CO.NH.(1H-tetrazol-5-yl) or a group of the formula **-CO.OR$^7$** or **-CO.NH.SO$_2$.R$^8$** in which $R^7$ is hydrogen or a non-toxic, biodegradable residue of a physiologically acceptable alcohol or phenol, and $R^8$ is (1-6C)alkyl, (3-8C)cycloalkyl or phenyl; and wherein any of said phenyl moieties may be unsubstituted or bear one or two substituents independently selected from (1-4C)alkyl, (1-4C)alkoxy, halogeno, cyano and trifluoromethyl; or an N-oxide thereof; or a non-toxic salt thereof.

It will appreciated that, depending on the nature of the substituents, certain of the formula I compounds may possess one or more chiral centres and may be isolated in one or more racemic or optically active forms. It is to be understood that this invention concerns any form of such a compound of formula I which possesses the afore-mentioned useful pharmacological properties, it being well known how to make optically active forms, for example by synthesis from suitable chiral intermediates, and how to determine their pharmacological properties, for example by use of the standard tests described hereinafter.

It is to be understood that generic terms such as "alkyl" include both straight and branched chain variants when the carbon numbers permit. However, when a particular radical such as "propyl" is given, it is specific to the straight chain variant, branched chain variants such as "isopropyl" being specifically named where intended. The same convention applies to other radicals.

A particular value for $R^1$, $R^3$ or $R^5$, when it is alkyl is, for example, (1-6C)alkyl such as methyl, ethyl, propyl, isopropyl, butyl, isobutyl, sec-butyl, pentyl or hexyl; and when it is cycloalkyl is, for example, cyclopropyl, cyclopentyl or cyclohexyl.

A particular value for $R^1$ or $R^3$ when it is alkyl bearing one or more fluoro substitutents is, for example, fluoromethyl, trifluoromethyl, 2,2,2-trifluoroethyl or pentafluoroethyl; and when it is alkyl bearing a cycloalkyl, (1-4C)alkoxy or phenyl substituent is, for example, cyclopropylmethyl, cyclopentylmethyl, cyclohexylmethyl, 2-methoxyethyl, 2-ethoxyethyl, benzyl, 1-phenylethyl or 2-phenylethyl.

A particular value for $R^2$ or $R^4$ when it is alkyl is, for example, methyl, ethyl, propyl, isopropyl or butyl; when it is alkoxycarbonyl is, for example, methoxycarbonyl, ethoxycarbonyl or propoxycarbonyl; when it is alkenyloxycarbonyl is, for example, allyloxycarbonyl, 2-methyl-2-propenyloxycarbonyl or 3-methyl-3-butenyloxycarbonyl; and when it is alkanoyl is, for example, formyl, acetyl or propionyl.

Particular values for $R^2$ or $R^3$ are, by way of example, for alkylamino: methylamino, ethylamino or butylamino; and for dialkylamino: dimethylamino, diethylamino or dipropylamino.

Particular values for $R^2$, $R^3$ or $R^4$, when it is halogeno is, for example, fluoro, chloro, bromo or iodo; and when it is alkoxy is, for example, methoxy or ethoxy;

Particular values for $R^2$ include, by way of example, for N-alkylcarbamoyl: N-methyl and N-ethylcarbamoyl; for di(N-alkyl)carbamoyl: N,N-dimethylcarbamoyl and N,N-diethylcarbamoyl; for alkanoylamino: formamido, acetamido and propanamido; for alkanoylamino: formamido, acetamido and propanamido; and for 3-alkylureido: 3-methylureido, 3-ethylureido and 3-propylureido

Particular values for $R^4$ or R5 include, by way of example, for alkyl containing one or more fluoro substituents: fluoromethyl, trifluoromethyl, 2,2,2-trifluoroethyl, pentafluoroethyl and heptafluoropropyl; for alkylthio: methylthio and ethylthio; for alkylsulphinyl: methylsulphinyl and ethylsulphinyl; and for alkylsulphonyl: methylsulphonyl and ethylsulphonyl.

A particular value for $R^5$ when it is cycloalkylalkyl is, for example, cyclopropylmethyl, cyclopentylmethyl, cyclohexylmethyl or 2-cyclopentylethyl; when it is phenylalkyl is, for example, benzyl, 1-phenylethyl or 2-phenylethyl; when it is alkoxycarbonyl is, for example, methoxycarbonyl, ethoxycarbonyl or propoxycarbonyl; when it is alkenyloxycarbonyl is, for example, allyloxycarbonyl, 2-methyl-2-propenyloxycarbonyl or 3-methyl-3-butenyloxycarbonyl; when it is alkanoyl is, for example, formyl, acetyl or propionyl; and when it is hydroxyalkyl is, for example, hydroxymethyl, 1-hydroxyethyl or 2-hydroxyethyl.

A particular value for $R^6$ when it is alkyl is, for example, methyl or ethyl; when it is halogeno is, for example, fluoro, chloro, bromo or iodo; and when it is alkoxy is, for example, methoxy or ethoxy.

A particular value for $R^7$ when it is a non-toxic, biodegradable residue of a physiologically acceptable alcohol or phenol is, for example, a residue derived from a (1-6C)alkanol such as methanol or ethanol, or phenol, glycerol or the like.

A particular value for $R^8$ when it is alkyl is, for example, methyl, ethyl, propyl, isopropyl, butyl or pentyl; and when it is cycloalkyl is, for example, cyclobutyl, cyclopentyl or cyclohexyl.

Particular values for optional substituents which may be present on phenyl moieties or for an optional substituent which may be present when X is phenylene include, by way of example, for halogeno: fluoro, chloro and bromo; for alkyl: methyl and ethyl; and for alkoxy: methoxy and ethoxy.

A particular value for an optional alkanoyl substituent on X which may be present when X is phenylene is, for example, formyl, acetyl or propionyl.

A specific value for X which is of particular interest is, for example, p-phenylene.

A preferred value for $R^1$ is, for example, (1-4C)alkyl such as methyl or ethyl.

A preferred value for $R^2$ is, for example, hydrogen.

A preferred value for $R^3$ is, for example, hydrogen, (1-4C)alkyl (such as methyl or ethyl), (1-4C)alkyl containing one or more fluoro substituents (such as trifluoromethyl), halogeno (such as chloro) or (1-4C)alkoxy (such as methoxy or ethoxy).

A preferred value for $R^4$ is, for example, hydrogen, formyl or halogeno (such as chloro).

A preferred value for $R^5$ is, for example, (1-4C)alkyl (such as methyl), alkoxycarbonyl (such as methoxycarbonyl) or cyano.

A preferred value for $R^6$ or $R^7$ is, for example, hydrogen.

A preferred value for Z is, for example, 1H-tetrazol-5-yl and which is especially preferred when attached ortho to the group X.

A preferred group of compounds of the formula I comprises compounds of the formula Ia wherein $R^1$, $R^2$, $R^3$, $R^4$, $R^5$ and $R^6$ have any of the meanings defined above; Ra is selected from hydrogen, (1-4C)alkyl, (1-4C)alkoxy, halogeno, (1-4C)alkanoyl, trifluoromethyl, cyano and nitro; and $Z^1$ is carboxy or 1H-tetrazol-5-yl; and the non-toxic salts thereof.

A particularly preferred sub-group of compounds of the formula Ia comprises those compounds in which

$R^1$ is (1-4C)alkyl; $R^2$ is hydrogen; $R^3$ is hydrogen, (1-4C)alkyl, (1-4C)alkoxy, halogeno or trifluoromethyl; $R^4$ is hydrogen, halogeno or formyl; $R^5$ is (1-4C)alkyl, (1-4C)alkoxycarbonyl or cyano; $R^6$ and Ra are both hydrogen; and $Z^1$ is 1H-tetrazol-5-yl; and the non-toxic salts thereof.

Compounds of the invention which are of particular interest include, for example, the specific embodiments set out hereinafter in the accompanying Examples and these are provided as a further feature of the invention. Of these compounds, those of Examples 1, 2, 4, 5, 6 and 8 are particularly preferred.

Although all of the formula I compounds can form salts with suitable acids, it will be appreciated that those compounds of formula I wherein Z is other than an ester group or in which $R^2$, $R^4$ or $R^5$ bear a carboxy group can form salts with bases as well as with acids. Particularly suitable non-toxic salts for such compounds therefore also include, for example, salts with bases affording physiologically acceptable cations, for example, alkali metal (such as sodium and potassium), alkaline earth metal (such as magnesium and calcium), aluminium and ammonium salts, as well as salts with suitable organic bases, such as with ethanolamine, methylamine, diethylamine or triethylamine, as well as salts with acids forming physiologically acceptable anions, such as salts with mineral acids, for example with hydrogen halides (such as hydrogen chloride and hydrogen bromide), sulphuric and phosphoric acid, and with strong organic acids, for example with p-toluenesulphonic and methanesulphonic acids.

The compounds of formula I may be obtained by standard procedures of organic chemistry well known in the art for the production of structurally analogous compounds. Such procedures are provided as a further feature of the invention and include, by way of example, the following procedures in which the generic radicals have any of the values given above, unless stated otherwise:

a) For those compounds in which Z is carboxy (that is in which Z is a group of the formula **-CO.OR$^7$** in which $R^7$ is hydrogen), a carboxylic acid derivative of the formula II, in which Q is a protected carboxy group selected from (1-6C)alkoxycarbonyl (especially methoxy-, ethoxy-, propoxy- or t-butoxy-carbonyl), phenoxycarbonyl, benzyloxycarbonyl and carbamoyl, is converted to carboxy.

The conversion may be carried out, for example by hydrolysis, conveniently in the presence of a suitable base such as an alkali metal hydroxide, for example, lithium, sodium or potassium hydroxide. The hydrolysis is generally carried out in the presence of a suitable aqueous solvent or diluent, for example in an aqueous (1-4C)alkanol, such as aqueous methanol or ethanol. However, it may also be performed in a mixture of an aqueous and non-aqueous solvent such as water and toluene using a conventional quaternary ammonium phase transfer catalyst. The hydrolysis is generally performed at a temperature in the range, for example, O - 12O°C, depending on the reactivity of the group Q. In general, when Q is carbamoyl, temperatures in the range, for example, 4O - 12O°C are required to effect the hydrolysis.

Alternatively, when Q is benzyloxycarbonyl the conversion may also be performed by hydrogenolysis, for example using hydrogen at 1-3 bar in the presence of a suitable catalyst, such as palladium on charcoal or on calcium sulphate, in a suitable solvent or diluent such as a (1-4C)alkanol (typically ethanol or 2-propanol) and at a temperature in the range, for example, O - 4O°C.

Further, when Q is t-butoxycarbonyl, the conversion may also be carried out by hydrolysis at a temperature in the range, for example, O - 1OO°C, in the presence of a strong acid catalyst, such as trifluoroacetic acid. The hydrolysis may either be performed in an excess of the acid or in the presence of a suitable diluent such as tetrahydrofuran, t-butyl methyl ether or 1,2-dimethoxyethane.

b) For those compounds of formula I wherein Z is tetrazolyl, a compound of the formula III in which L is a suitable protecting group, such as trityl, benzhydryl, trialkyltin (for example trimethyltin or tributyltin) or triphenyltin, affixed to a nitrogen of the tetrazolyl moiety, is deprotected.

The reaction conditions used to carry out the deprotection necessarily depend on the nature of the group L. As an illustration, when it is trityl, benzhydryl, trialkyltin or triphenyltin, the decomposition conditions include, for example, acid catalysed hydrolysis in a mineral acid (such as aqueous hydrochloric acid), conveniently in an aqueous solvent (such as aqueous dioxan or 2-propanol). Alternatively, a trityl or benzhydryl group may be removed by hydrogenolysis, for example as described in (a) above for conversion of a benzyloxycarbonyl to a carboxy.

Compounds of the formula III wherein L is trialkyltin or triphenyltin may be obtained, for example, by reaction of a nitrile of the formula VII with a trialkyltin azide, such as tributyltin azide, or triphenyltin azide respectively. The reaction is conveniently carried out in a suitable solvent or diluent, such as toluene or xylene, and at a temperature in the range, for example, 5O-15O°C. In a modified procedure, a formula I compound wherein Z is tetrazolyl may be obtained directly by in situ removal of the trialkyltin or triphenyltin group without prior isolation of the formula III compound, for example by the addition of aqueous mineral acid or gaseous hydrogen chloride to the reaction mixture. The nitriles of the formula VII may be obtained, for example, by alkylation of a compound of the formula IV with a nitrile of the formula VIII wherein **Hal.** stands for a suitable leaving group such as chloro, bromo, iodo, methanesulphonyloxy or p-toluenesulphonyloxy, using similar conditions to those used in process (c) described hereinafter. The necessary compounds of formula VIII may be made by standard procedures such as that illustrated in Scheme 1, using methods of organic chemistry well known in the art. Alternatively, the nitriles of the formula VII may be obtained from stepwise conversion of a compound of formula I wherein Z is a group of the formula $-CO.OR^7$ under standard conditions. Trialkyltin azides and triphenyltin azides are either commercially available or may be prepared by standard procedures well known in the art, such as by reaction of a trialkyltin halide with an alkali metal azide.

c) A compound of the formula IV is alkylated with a compound of the formula V wherein **Hal.** stands for a suitable leaving group such as chloro, bromo, iodo, methanesulphonyloxy or p-toluenesulphonyloxy.

The reaction is generally carried out in the presence of a suitable base, for example, an alkali metal alkoxide such as sodium methoxide or sodium ethoxide or an alkali metal hydride such as sodium hydride or an alkali metal carbonate such as sodium or potassium carbonate, or an organic base such as diisopropylethylamine and in a suitable solvent or diluent, for example, a (1-4C)alkanol such as methanol or ethanol when an alkali metal alkoxide is used, or in a polar solvent such as N,N-dimethylformamide or N-methylpyrrolidone and at a temperature in the range, for example, 1O - 1OO°C. Alternatively, a quaternary ammonium hydroxide may be used in a mixture of an aqueous and non-aqueous solvent such as water and dichloromethane. In carrying out process (c), when in the starting material Z is an acidic group, about two molecular equivalents of a suitable base is generally required, whereas when Z is a non-acidic group the presence of one molecular equivalent of a suitable base is generally sufficient.

Procedure (c) is particularly suitable for the production of those compounds of the formula I in which Z is a group of the formula $\mathbf{-CO.OR^7}$ in which $R^7$ is other than hydrogen, for example wherein $R^7$ is (1-6C)alkyl, benzyl or phenyl, which compounds are also starting materials of formula II for the reaction described in (a) above. Similarly, using an analogous procedure, but starting with the appropriate halomethyl tetrazolyl derivative of the formula VI, the starting materials of the formula III may be obtained for procedure (b).

Certain of the compounds of formula IV are already known and the remainder can be made by analogy therewith using standard procedures of organic chemistry well known in the art, for example as described in standard works of heterocyclic chemistry such as that edited by Elderfield or as illustrated in Schemes 2 to 4. The compounds may also be obtained by using similar procedures to those well known in the art for the preparation of the structurally analogous indoles but starting from an appropriately substituted pyridine derivative.

The necessary compounds of the formula V (and also of formula VI) may be made by standard procedures such as those which are illustrated in Scheme 1 for compounds in which X is phenylene.

Compounds of the formula VI wherein X is phenylene may also be conveniently obtained by reaction of a Grignard reagent, formed from a suitably substituted 4-bromotoluene, with a trialkyltin halide, such as tributyltin chloride, followed by reaction of the resulting (substituted)phenyltrialkyltin compound with a bromobenzonitrile in the presence of a palladium(O) catalyst, such as tetrakis(triphenylphosphine)palladium, and azo(bisisobutyronitrile). The resultant substituted 41-methyl-biphenylcarbonitrile may then be converted to a compound of the formula VI by carrying out steps (b), (c) and (d) in a similar manner to that shown in Scheme 1. Alternatively, suitably substituted 41-methylbiphenylcarbonitriles may be obtained by reaction of 4-methylphenylboronic acid with an appropriately substituted bromobenzonitrile in the presence of a suitable palladium catalyst, such as palladium (II)chloride or tetrakis(triphenylphosphine)palladium, and azo(bisisobutyronitrile).

Whereafter, those compounds of formula I wherein Z is 1H-tetrazol-5-yl may be obtained by stepwise conversion of a compound of the formula I wherein Z is a group of the formula $-CO.OR^7$ into the corresponding nitrile under standard conditions, followed by reaction of the nitrile with an azide such as an alkali metal azide, preferably in the presence of an ammonium halide, and preferably in the presence of a suitable polar solvent such as N,N-dimethylformamide and at a temperature in the range, for example, 5O to 16O°C.

Whereafter, a compound of the formula I wherein $R^3$ is hydrogen may be obtained by catalytic hydrogenation of a compound of the formula I in which $R^3$ is halogeno (such as chloro), for example by hydrogenation over a suitable catalyst such as palladium on carbon at 1-3 bar in a suitable solvent or diluent such as an (1-4C)alkanol (for example ethanol), and at a temperature in the range, for example, O-4O°C.

Whereafter, those compounds of the formula I wherein Z is -CO.NH.(1H-tetrazol-5-yl), a group of the formula $\mathbf{-CO.NH.SO_2R^8}$ or a group of the formula $\mathbf{-CO.OR^7}$ in which $R^7$ is other than hydrogen, may be obtained, for example, by reacting a carboxylic acid of the formula I in which Z is carboxy (or a reactive derivative of said acid) with 5-aminotetrazole, a sulphonamide of the formula $\mathbf{NH_2.SO_2R^8}$ or a salt thereof (for example, an alkali metal salt), or a hydroxy compound of the formula $\mathbf{HO.R^7}$ or with a salt thereof (for example, an alkali metal thereof). Suitable reactive derivatives include, for example the chloride, bromide, azide, anhydride and mixed anhydride with formic or acetic acid of the carboxylic acid of formula I as defined above. When the free acid form is used, the reaction is generally carried out in the presence of a suitable dehydrating agent such as dicyclohexycarbodiimide or 3-(3-dimethylaminopropyl)-1-ethylcarbodiimide in the presence of a base such as

triethylamine, pyridine or 4-dimethylaminopyridine. When a reactive derivative is used, either the reaction is carried out in the presence of a base such as mentioned above, or, for the preparation of a compound of the formula I wherein Z is a group of the formula **$-CO.NH-SO_2R^8$** or a group of the formula **$-CO.OR^7$**, the sulphonamide or hydroxy compound is used in the form of a salt, such as its alkali metal salt (in particular the lithium, sodium or potassium salt thereof). The reaction is generally performed in the presence of a suitable diluent or solvent such as dioxan, t-butyl methyl ether or tetrahydrofuran and at a temperature in the range, for example, O - 6O°C.

Whereafter, when an N-oxide derivative of a compound of the formula I is required, a compound of the formula I is oxidised. Suitable oxidising agents include those well known in the art for the conversion of nitrogen heterocycles to their corresponding N-oxide derivatives, for example, hydrogen peroxide or an organic peracid such as m-chloroperbenzoic acid or peracetic acid. The oxidation is preferrably carried out in a suitable conventional solvent or diluent for such oxidations, for example dichloromethane, chloroform or acetic acid, and at a temperature in the general range, for example O to 8O°C.

Whereafter, when a non-toxic salt of a compound of formula I is required, it may be obtained, for example, by reaction with the appropriate base affording a physiologically acceptable cation, or with the appropriate acid affording a physiologically acceptable anion, or by any other conventional salt formation procedure.

Further, when an optically active form of a compound of formula I is required, one of the aforesaid processes may be carried out using an optically active starting material. Alternatively, the racemic form of a compound of formula I in which Z is an acidic group may be resolved, for example by reaction with an optically active form of a suitable organic base, for example, ephedrine, N,N,N-trimethyl-(1-phenylethyl)ammonium hydroxide or 1-phenylethylamine, followed by conventional separation of the diastereoisomeric mixture of salts thus obtained, for example by fractional crystallisation from a suitable solvent, for example a (1-4C)alkanol, whereafter the optically active form of said compound of formula I may be liberated by treatment with acid using a conventional procedure, for example using an aqueous mineral acid such as dilute hydrochloric acid.

According to a further aspect of the invention, there is provided a process for the manufacture of a compound of the formula I wherein Z is tetrazolyl, X is p-phenylene optionally bearing a substituent selected from (1-4C)alkyl, (1-4C)alkoxy, halogeno, (1-4C)alkanoyl, trifluoromethyl, cyano and nitro, and $R^1$, $R^2$, $R^3$, $R^4$ $R^5$ and $R^6$, have any of the meanings defined hereinbefore; which comprises reaction of a compound of the formula IX wherein $P^1$ is an electron-deficient phenyl group; Ra is hydrogen, (1-4C)alkyl, (1-4C)alkoxy, halogeno, (1-4C)alkanoyl, trifluoromethyl, cyano or nitro; and $R^1$, $R^2$, $R^3$ , $R^4$, $R^5$ and $R^6$ have any of the values defined above with a base selected from an alkali metal hydroxide, (1-12C)alkanolate, (1-12C)alkanethiolate, phenolate, thiophenolate or diphenylphosphide, wherein any phenyl ring of the latter three groups may optionally bear a (1-4C)alkyl, (1-4C)alkoxy or halogeno group.

A particular value for $P^1$ includes, for example, a phenyl group bearing 1, 2 or 3 electron-withdrawing groups independently selected from nitro, cyano and trifluoromethyl.

A particular value for Ra when it is alkyl is, for example, methyl or ethyl; when it is alkoxy is, for example, methoxy or ethoxy; when it is alkanoyl is, for example, formyl, acetyl or propionyl; and when it is halogeno is, for example, fluoro, chloro, bromo or iodo.

A particular value for a base includes the following by way of example:-

for an alkali metal hydroxide: sodium or potassium hydroxide;

for an alkali metal alkanolate: an alkali metal (1-8C)alkanolate, for example an alkali metal (1-4C)alkoxide, such as sodium or potassium methoxide, ethoxide, propoxide or butoxide;

for an alkali metal alkanethiolate: an alkali metal
(1-8C)alkanethiolate, for example an alkali metal (1-4C)alkanethiolate such as sodium or potassium methanethiolate, ethanethiolate, propanethiolate or butanethiolate.

A particular value for an optional substituent on a phenyl group of an alkali metal phenolate, thiophenolate or diphenylphosphide, when it is alkyl is, for example, methyl or ethyl; when it is alkoxy is, for example, methoxy or ethoxy; and when it is halogeno is, for example, fluoro, chloro or bromo.

A preferred value for $P^1$ is, for example, a nitrophenyl group, especially 4-nitrophenyl.

A preferred value for X is, for example, when it is unsubstituted p-phenylene.

A particularly preferred base is an alkali metal alkanethiolate such as sodium or potassium propanethiolate, an alkali metal alkanolate such as sodium or potassium ethoxide or methoxide, or an alkali metal thiophenolate such as sodium or potassium 4-fluorothiophenolate.

It will be appreciated that when the base is an alkali metal alkanolate, alkanethiolate, phenolate, thiophenolate or diphenylphosphide, it may be generated in situ from the corresponding alkanol, alkanethiol, phenol, thiophenol or diphenylphosphine with a suitable alkali metal base such as an alkali metal hydride, for example, lithium, potassium or sodium hydride.

The process of the invention is particularly useful for the preparation of compounds of the formula I wherein

the tetrazolyl group is at the ortho position relative to the adjacent phenyl group.

The reaction is conveniently carried out in a suitable inert organic solvent or diluent, for example, a polar solvent such as N,N-dimethylformamide or N-methylpyrrolidone. Alternatively, an alkanol such as methanol or ethanol may be used, for example, when an alkali metal hydroxide or alkoxide such as sodium or potassium hydroxide, methoxide or ethoxide is employed. The reaction is generally carried out at a temperature in the range, for example, -30°C to 50°C. It will be appreciated that the choice of temperature will depend on the nature of the base employed. For example, when an alkali metal alkanethiolate or alkanolate is used, a temperature in the range of 0°C to ambient temperature is preferred.

Compounds of the formula IX may be obtained by reaction of a boronic acid of the formula X with a compound of the formula XI wherein $P^1$ is an electron-deficient phenyl group having any of the meanings defined above and W is a bromo, iodo or trifluoromethanesulphonyloxy group, in the presence of a palladium(O) catalyst, such as tetrakis(triphenylphosphine)palladium, and azo(bisisobutyronitrile). The reaction is preferably carried out in the presence of a base, such as sodium or potassium carbonate, in an inert solvent or diluent, for example, a hydrocarbon such as toluene or xylene, an ether, such as dioxan or tetrahydrofuran, an (1-4C)alkanol such as methanol or ethanol, water, or mixture thereof, for example a mixture of water, methanol and toluene, and at a temperature in the range of, for example, 50°C to 150°C., and conveniently at or about the reflux temperature of the solvent or mixture of solvents used.

Compounds of the formula X may be obtained, for example, by heating at reflux a 4-methylphenylboronic acid in a solvent such as methyl chloroform with azeotropic removal of water, followed by radical bromination of the product which may be carried out in situ, for example with bromine or N-bromosuccinimide in the presence of azo(bisisobutyronitrile). The resultant 4-bromomethylphenylboronic acid anhydride may then be used to alkylate a compound of the formula IV (using similar alkylation conditions to those used in process (c) described above), followed by subsequent acidic hydrolysis, to give a formula X compound. Alternatively the product from the alkylation step prior to hydrolysis may be isolated and reacted directly with a compound of the formula XI under similar conditions to those described above to obtain a formula IX compound directly. In a yet further alternative procedure, a 4-methylphenylboronic acid and an appropriate alkanediol, for example 2,2-dimethylpropan-1,3-diol, may be heated at reflux in a solvent (such as cyclohexane) with azeotropic removal of water followed by free radical bromination of the product, which may be carried out in situ. The resultant bromomethyl compound may then be reacted using analogous procedures to those described above for the 4-bromomethylphenylboronic acid anhydride to obtain a formula X compound or a compound of the formula IX directly. Compounds of the formula XI may be obtained, for example, as shown in Scheme 5.

Whereafter, an N-oxide or a non-toxic salt or an optically active form of a compound of the formula I may be obtained as described above if desired.

Certain of the intermediates defined herein are novel, for example the compounds of the formula II, III, IV, VII and IX and are provided as a further feature of the invention.

As stated above, the compounds of formula I will have beneficial pharmacological effects in warm-blooded animals (including man) in diseases and medical conditions where amelioration of the vasoconstrictor and fluid retaining properties of the renin-angiotensin-aldosterone system is desirable, at least in part by antagonism of one or more of the physiological actions of AII. The compounds of the invention will thus be useful in the treatment of diseases or medical conditions such as hypertension, congestive heart failure and/or hyperaldosteronism in warm-blooded animals (including man), as well as in other diseases or medical conditions in which the renin-angiotensin-aldosterone system plays a significant causative role. The compounds of the invention may also be useful for the treatment of ocular hypertension, glaucoma, cognitive disorders (such as Alzheimer's disease, amnesia, senile dementia and learning disorders), as well as other diseases such as renal failure, cardiac insufficiency, post-myocardial infarction, cerebrovascular disorders, anxiety, depression and certain mental illnesses such as schizophrenia.

The antagonism of one or more of the physiological actions of AII and, in particular, the antagonism of the interaction of AII with the receptors which mediate its effects on a target tissue, may be assessed using one or more of the following, routine laboratory procedures:

Test A: This in vitro procedure involves the incubation of the test compound initially at a concentration of 100 micromolar (or less) in a buffered mixture containing fixed concentrations of radiolabelled AII and a cell surface membrane fraction prepared from a suitable angiotensin target tissue. In this test, the source of cell surface membranes is the guinea pig adrenal gland which is well known to respond to AII. Interaction of the radiolabelled AII with its receptors (assessed as radiolabel bound to the particulate membrane fraction following removal of unbound radiolabel by a rapid filtration procedure such as is standard in such studies) is antagonized by compounds which also bind to the membrane receptor sites and the degree of antagonism (observed in the test as displacement of membrane-bound radioactivity) is determined readily by comparing the receptor-bound radioactivity in the presence of the test compound at the specified test concentration with a control value determined in the absence of the test compound. Using this procedure compounds showing at least 50% displacement of radiolabelled AII binding at a concentration of $10^{-4}$ M are retested at lower concentrations to determine their potency. For determination of the $IC_{50}$ (concentration for 50% displacement of radiolabelled AII binding), concentrations of the test compound are ordinarily chosen to allow testing over at least four orders of magnitude centred about the predicted approximate $IC_{50}$, which latter is subsequently determined from a plot of percentage displacement against concentration of the test compound.

In general, acidic compounds of formula I as defined above show significant inhibition in **Test A** at a concentration of 50 micromolar or much less.

Test B: This in vitro test involves the measurement of the antagonistic effects of the test compound against AII-induced contractions of isolated rabbit aorta, maintained in a physiological salt solution at 37°C. In order to ensure that the effect of the compound is specific to antagonism of AII, the effect of the test compound on noradrenaline-induced contraction may also be determined in the same preparation.

In general, acidic compounds of formula I as defined above show significant inhibition in **Test B** at a final concentration of 50 micromolar or much less. [Note: Compounds of formula I wherein Z is a group of the formula **-CO.OR$^7$** in which $R^7$ is other than hydrogen in general show only weak activity in the in vitro **Tests A or B.**]

Test C: This in vivo test involves using terminally-anaesthetised or conscious rats in which an arterial catheter has been implanted under anaesthesia for the measurement of changes in blood pressure. The AII antagonistic effects of the test compound following oral or parenteral administration, are assessed against angiotensin II-induced pressor responses. To ensure that the effect is specific, the effect of the test compound on vasopressin-induced pressor responses may also be determined in the same preparation.

The compounds of formula I generally show specific AII-antagonist properties in **Test C** at a dose of 50 mg/kg body weight or much less, without any overt toxicological or other untoward pharmacological effect.

Test D: This in vivo test involves the stimulation of endogenous AII biosynthesis in a variety of species including rat, marmoset and dog by introducing a diet of low sodium content and giving appropriate daily doses of a saluretic known as frusemide. The test compound is then administered orally or parenterally to the animal in which an arterial catheter has been implanted under anaesthesia for the measurement of changes in blood pressure.

In general compounds of formula I will show AII-antagonist properties in **Test D** as demonstrated by a significant reduction in blood pressure at a dose of 50 mg/kg body weight or much less, without any overt toxicological or other untoward pharmacological effect.

By way of illustration of the angiotensin II inhibitory properties of compounds of the formula I, the compound of Example 2 gave the following results in tests A and C described above:-

In test A: an $IC_{50}$ of $7.5\times10^{-8}$ M;

In test C: an $ED_{50}$ of 0.4 mg/kg (i.v. administration).

The compounds of formula I will generally be administered for therapeutic or prophylactic purposes to warm-blooded animals (including man) requiring such treatment in the form of a pharmaceutical composition, as is well known in the pharmaceutical art. According to a further feature of the invention there is provided a pharmaceutical composition comprising a compound of formula I, or a salt or N-oxide thereof as defined above, together with a pharmaceutically acceptable diluent or carrier. Such compositions will conveniently be in a form suitable for oral administration (e.g. as a tablet, capsule, solution, suspension or emulsion) or parenteral administration (e.g. as an injectable aqueous or oily solution, or injectable emulsion).

The compounds of formula I may also be advantageously administered for therapeutic or prophylactic purposes together with another pharmacological agent known in the general art to be of value in treating one or more of the diseases or medical conditions referred to hereinabove, such as a beta-adrenergic blocker (for example atenolol), a calcium channel blocker (for example nifedipine), an angiotensin converting enzyme (ACE) inhibitor (for example lisinopril) or a diuretic (for example furosemide or hydrochlorothiazide). It is to be understood that such combination therapy constitutes a further aspect of the present invention.

In general a compound of formula I (or a pharmaceutically acceptable salt thereof as appropriate) will generally be administered to man so that, for example, a daily oral dose of up to 50 mg/kg body weight (and preferably of up to 10 mg/kg) or a daily parenteral dose of up to 5 mg/kg body weight (and preferably of up to 1 mg/kg) is received, given in divided doses as necessary, the precise amount of compound (or salt) administered and the route and form of administration depending on size, age and sex of the person being treated and on the particular disease or medical condition being treated according to principles well known in the medical arts.

In addition to their aforesaid use in therapeutic medicine in humans, the compounds of formula I are also useful in the veterinary treatment of similar conditions affecting commercially valuable warm-blooded animals, such as dogs, cats, horses and cattle. In general for such treatment, the compounds of the formula I will generally be administered in an analogous amount and manner to those described above for administration to humans. The compounds of formula I are also of value as pharmacological tools in the development and standardisation of test systems for the evaluation of the effects of AII in laboratory animals such as cats, dogs, rabbits, monkeys, rats and mice, as part of the continuing search for new and improved therapeutic agents.

The invention will now be illustrated by the following non-limiting Examples in which, unless otherwise stated:-

(i) concentrations and evaporations were carried out by rotary evaporation in vacuo;

(ii) operations were carried out at room temperature, that is in the range 18-26°C;

(iii) flash column chromatography was performed on Merck Kieselgel 60 (Art. no. 9385) obtained from E Merck, Darmstadt, Germany;

(iv) yields, where given, are intended for the assistance of the reader only and are not necessarily the maximum attainable by diligent process development;

(v) $^1H$ NMR spectra were normally determined at 200 MHz in $CDCl_3$ using tetramethylsilane (TMS) as an internal standard, and are expressed as chemical shifts (delta values) in parts per million relative to TMS using conventional abbreviations for designation of major peaks: s, singlet; m, multiplet; t, triplet; br, broad; d,doublet;

(vi) $^{13}C$ NMR spectra were normally determined at 100 MHz in $CDCl_3$ or $d_6$-dimethylsulphoxide ($d_6$-DMSO) using the solvent signal as internal standard, and are expressed as chemical shifts (delta values) in parts per million relative to TMS;

(vii) the purity of chemical products was assessed by nuclear magnetic resonance spectroscopy, thin layer chromatographic analysis and/or microanalysis: and

(viii) the term "1H-tetrazol-5-yl" stands for "1H-1,2,3,4-tetrazol-5-yl".

## Example 1

A mixture of 3,4-dichloro-6-ethyl-2-methyl-1-[(2′-(2-triphenylmethyl-2H-tetrazol-5-yl)biphenyl-4-yl)methyl]-1H-pyrrolo-[3,2-c]pyridine (A) (380 mg), methanol (4 ml) and concentrated hydrochloric acid (0.2 ml) was stirred for 5 hours. Volatile material was removed by evaporation and the residue was triturated with ether (30 ml). The solid was collected by filtration, washed with ether (25 ml) and recrystallised from methanol/ether to give 3,4-dichloro-6-ethyl-2-methyl-1-[(2′-(1H-tetrazol-5-yl)biphenyl-4-yl)methyl]-1H-pyrrolo[3,2-c]pyridine hydrochloride (250 mg), as a white amorphous solid; NMR ($d_6$-DMSO): 1.23(t,3H), 2.31(s,3H), 2.77(q,2H), 5.51(s,2H), 6.96(d,2H), 7.06(d,2H), 7.45-7.70(m,5H); mass spectrum [positive fast atom bombardment (+ve FAB), DMSO/m-nitrobenzyl alcohol (NBA)]: 463 $(M+H)^+$; microanalysis, found: C,57.0; H,4.3; N,16.6; Cl,20.0; $H_2O$,1.7%; $C_{24}H_2O\ Cl_2N_6.HCl.0.5H_2O$ requires: C,56.6; H,4.3; N,16.5; Cl,20.9; $H_2O$,1.8%.

The starting material (A) was obtained as follows:-

(i) A mixture of 2,4-dihydroxy-6-ethylpyridine (obtained as described in European Patent Application, Publication No. 332030) (12 gm) and hydrazine hydrate (43 ml) in 2-ethoxyethanol (210 ml) was refluxed for 48 hours. Further hydrazine hydrate (10 ml) was then added and the mixture refluxed for a further 21 hours. Volatile material was removed by evaporation and water (50 ml) was added to give a solid residue. The solid was collected by filtration and added to methanol (100 ml). The mixture was evaporated and the residue was dissolved in a mixture of methanol (50 ml) and ethanol (100 ml) by heating on a steam-bath. Acetone (35 ml) was added to the hot solution and the mixture was refluxed for 3 hours. Volatile material (30 ml) was then removed by evaporation and the residual liquid cooled in an ice-water bath. The solid which crystallised was collected by filtration, washed with ethanol and air dried to give 6-ethyl-4-isopropylidenehydrazino-1H-pyridin-2-one (10 gm), m.p. 252-257°C (dec.); NMR ($d_6$-DMSO): 1.12(t,3H), 1.66(s,3H), 1.93(s,3H), 2.38(q,2H), 5.56(d,1H), 5.88(s,1H), 9.0(s,1H), 10.62(brs,1H).

(ii) A stirred mixture of 6-ethyl-4-isopropylidinehydrazino-1H-pyridin-2-one (10 gm) in a mixture of 26.5% diphenyl and 73.5% diphenyl ether (130 ml) was refluxed for 14 hours under an atmosphere of argon. The mixture was then cooled and the brown crystalline solid which separated was collected by filtration and washed with hexane. There was thus obtained 6-ethyl-2-methyl-4,5-dihydro-1H-pyrrolo[3,2-c]pyridin-4-one (8.2 gm) as a solid, m.p. 285-290°C (dec.); NMR ($d_6$-DMSO): 1.16(t,3H), 2.26(s,3H), 2.48(q,2H), 6.05(s,1H), 6.07(s,1H), 10.55(brs,1H), 11.0(brs,1H).

(iii) A mixture of 6-ethyl-2-methyl-4,5-dihydro-1HH-pyrrolo[3,2-c]pyridin-4-one (1.3 gm), phosphorus oxychloride (25 ml) and phosphorus pentachloride (1.55 gm) was heated on a steam-bath for 4 hours. Volatile material was then removed by evaporation. Ice was added to the residue and the mixture was basified with saturated aqueous sodium carbonate solution to pH8. The mixture was extracted with dichloromethane (2 x 45 ml) and the combined extracts were washed with saturated sodium chloride solution and dried ($MgSO_4$). The solvent was removed by evaporation to give a dark brown oil. The oil was purified by flash chromatography eluting with a mixture of methanol and dichloromethane (2:98 v/v) to give 3,4-dichloro-6-ethyl-2-methyl-1H-pyrrolo[3,2-c]pyridine as an amorphous solid (200 mg); NMR ($d_6$-DMSO): 1.22(t,3H),

2.36(s,3H), 2.75(q,2H), 7.15(s,1H), 11.94(brs,1H); mass spectrum (positive chemical ionisation (+ve CI)): 229$(M+H)^+$.

(iv) Sodium hydride (6O% dispersion in mineral oil; 35 mg) was added to a stirred solution of 3,4-dichloro-6-ethyl-2-methyl-1H-pyrrolo[3,2-c]pyridine (19O mg) in N,N-dimethylformamide (DMF) (3 ml). The mixture was stirred until evolution of hydrogen had ceased and then a solution of 5-[2-(4′-bromomethylbiphenylyl)]-2-triphenylmethyl-2H-tetrazole (obtained as described in European patent application, publication no. O291969) (51O mg) in DMF (3 ml) was added. The mixture was stirred for a further 3.5 hours. The solvent was removed by evaporation and the residue partitioned between water (2O ml) and ethyl acetate (6O ml). The organic layer was washed with saturated sodium chloride solution (2O ml) and dried ($MgSO_4$). The solvent was removed by evaporation and the resultant oil was purified by flash chromatography, eluting with ethyl acetate/dichloromethane (5:95 v/v) to give 3,4-dichloro-6-ethyl-2-methyl-1-[(2′-(2-triphenylmethyl-2H-tetrazol-5-yl)biphenyl-4-yl)methyl]-1H-pyrrolo[3,2-c]pyridine (A) (4OO mg) as a white solid, m.p. 188-19O°C (dec.); NMR ($d_6$-DMSO): 1.18(t,3H), 2.15(s,3H), 2.68(q,2H), 5.42(s,2H), 6.8-6.9(m,8H), 7.O5(d,2H), 7.2-7.45(m,11H), 7.45-7.65(m,2H), 7.8(dd,1H); mass spectrum [+ve FAB, DMSO/NBA]: 7O5 $(M+H)^+$.

## Example 2

Using an analogous procedure to that described in Example 1 but starting from 4-chloro-2,6-dimethyl-1-[(2′-(2-triphenylmethyl-2H-tetrazol-5-yl)biphenyl-4-yl)methyl]-1H-pyrrolo[3,2-c]pyridine (A), there was obtained 4-chloro-2,6-dimethyl-1-[(2′-(1H-tetrazol-5-yl)biphenyl-4-yl)methyl]-1H-pyrrolo[3,2-c]pyridine hydrochloride as a powder after recrystallisation from isopropanol; NMR ($d_6$-DMSO): 2.35(s,3H), 2.53(s,3H), 6.5(s,1H), 6.94(d,2H), 7.O6(d,2H), 7.46-7.6O(m,3H), 7.63(s,1H), 7.68(dd,1H); mass spectrum (+ve FAB, DMSO/NBA): 415 $(M+H)^+$; microanalysis, found C,61.O; H,4.8; N,18.O%; $C_{23}H_{19}N_6Cl.HCl.O.33$(isopropanol) requires: C,61.2; H,4.7; N,18.2%.

The starting material (A) was obtained as a gum using an analogous procedure to that described in Example 1; NMR ($d_6$-DMSO): 2.2(s,3H), 2.36(s,3H), 5.36(s,2H), 6.35(s,1H), 6.8-6.84(m,8H), 7.O3(d,2H), 7.2O-7.44(m,11H), 7.49-7.6O(m,2H), 7.79(dd,1H); mass spectrum (+ve FAB, DMSO/NBA): 657$(M+H)^+$; starting from 4-chloro-2,6-dimethyl-1H-pyrrolo[3,2-c]pyridine, itself obtained using the method described in Tetrahedron, 1976, 32, 1383-139O.

## Example 3

A solution of 4-chloro-2,6-dimethyl-1-[(2′-(1H-tetrazol-5-yl)biphenyl-4-yl)methyl]-1H-pyrrolo[3,2-c]pyridine hydrochloride (3OO mg) in ethanol (1O ml) was catalytically hydrogenated over 1O% palladium on carbon (75 mg) for 6.5 hours. The catalyst was removed by filtration through diatomaceous earth and the filtrate was concentrated by evaporation. The residue was triturated with ethyl acetate to give 2,6-dimethyl-1-[(2′-(1H-tetrazol-5-yl)biphenyl-4-yl)methyl]-1H-pyrrolo[3,2-c]pyridine hydrochloride (89 mg), as a white amorphous solid, m.p. 259-261°C (dec); NMR ($d_6$-DMSO): 2.42(s, 3H), 2.72(s, 3H), 5.6O(s, 2H), 6.61(s, 1H), 6.99(d, 2H), 7.O8(d, 2H), 7.45-7.72(m, 4H), 8.O2(s, 1H), 9.O1(s, 1H), 13.2(br s, 1H); mass spectrum (+ve FAB, DMSO/NBA): 381 $(M+H)^+$ ; microanalysis, found: C,64.6; H,5.3; N,19.5%; $C_{23}H_{20}N_6.HCl.O.5H_2O$ requires: C,64.9; H,5.2; N,19.7%.

## Example 4

Using an analogous procedure to that described in Example 1, but starting from 4-chloro-6-ethyl-2-methyl-1-[(2′-(2-triphenylmethyl-2H-tetrazol-5-yl)biphenyl-4-yl)methyl]-1H-pyrrolo[3,2-c]pyridine (A), there was thus obtained 4-chloro-6-ethyl-2-methyl-1-[(2′-(1H-tetrazol-5-yl)biphenyl-4-yl)methyl]-1H-pyrrolo[3,2-c] pyridine hydrochloride as a white solid after recrystallisation from ethanol/ethyl acetate; NMR ($d_6$-DMSO): 1.25(t, 3H), 2.35(s, 3H), 2.82(q, 2H), 5.5(s, 2H), 6.49(s, 1H), 6.94(d, 2H), 7.O6(d, 2H), 7.45-7.8(m, 5H); mass spectrum (+ve FAB, DMSO/NBA): 429 $(M+H)^+$ ; microanalysis, found: C, 62.1; H, 4.6; N, 18.3%; $C_{24}H_{21}N_6Cl.HCl$ requires C, 61.9; H, 4.8; N, 18.1%.

The starting material (A) was obtained as an amorphous solid using an analogous procedure to that described in Example 1, part (iv); NMR ($d_6$-DMSO): 1.16(t, 3H), 2.2(s, 3H), 2.66(q, 2H), 5.38(s, 2H), 6.38(s, 1H), 6.83(m, 8H), 7.O4(d, 2H), 7.2-7.46(m, 11H), 7.57(m, 2H), 7.79(dd, 1H); starting from 4-chloro-6-ethyl-2-methyl-1H-pyrrolo[3,2-c]pyridine, itself obtained as follows:-

(i) A mixture of 6-ethyl-2-methyl-4,5-dihydro-1H-pyrrolo[3,2-c]pyridin-4-one (8 g) and freshly distilled phosphorus oxychloride (8O ml) was heated under reflux for 2 hours. Volatile material was then removed by

evaporation. Ice was added to the residue and the mixture was basified with saturated aqueous sodium carbonate solution to pH 8. The mixture was extracted with dichloromethane (2 x 2OO ml) and the combined extracts were washed with saturated sodium chloride solution and dried ($MgSO_4$). The solvent was removed by evaporation to give a brown solid. The solid was heated with ethyl acetate (4O ml) and the hot mixture was filtered. The filtrate was evaporated to give 4-chloro-6-ethyl-2-methyl-1H-pyrrolo[3,2-c]pyridine as an amorphous solid (4.5 g); NMR ($d_6$-DMSO): 1.23(t, 3H), 2.39(s, 3H), 2.74(q, 2H), 6.15(s, 1H), 7.O9(s, 1H), 11.51(br s, 1H); mass spectrum (positive chemical ionisation (+ve CI)): 195$(M+H)^+$.

## Example 5

Using an analogous procedure to that described in Example 1, but starting from 4-chloro-6-ethyl-3-formyl-2-methyl-1-[(2′-(2-triphenylmethyl-2H-tetrazol-5-yl)biphenyl-4-yl)methyl]-1H-pyrrolo[3,2-c]pyridine (A), there was obtained 4-chloro-6-ethyl-3-formyl-2-methyl-1-[(2′-(1H-tetrazol-5-yl)biphenyl-4-yl)methyl]-1H-pyrrolo[3,2-c]pyridine hydrochloride as a solid (after trituration with ethyl acetate); NMR ($d_6$-DMSO): 1.25(t, 3H), 2.7O(s, 3H), 2.8O(q, 2H), 5.6O(s, 2H), 6.8O(d, 2H), 7.O8(d, 2H), 7.4O-7.7O(m, 5H), 1O.69(s, 1H); mass spectrum (+ve FAB, DMSO/NBA): 457$(M+H)^+$.

The starting material (A) was obtained as follows:-

(i) DMF (2 ml) cooled to -5°C under an atmosphere of argon and phosphorus oxychloride (O-68 ml) was added. The mixture was stirred for 5 minutes and then a solution of 4-chloro-6-ethyl-2-methyl-1H-pyrrolo[3,2-c]pyridine (1.3 g) in DMF (1.5 ml) was added dropwise. The mixture was stirred for O.5 hour at ambient temperature and then heated at 45°C for 6 hours. After cooling, the mixture was poured onto ice and adjusted to pH6 by adding 2M aqueous sodium hydroxide solution. The mixture was extracted with dichloromethane (2 x 8O ml) and the combined extracts were washed with saturated sodium chloride solution and dried ($MgSO_4$). The solvent was removed by evaporation to give a brown gum which was purified by flash chromatography, eluting with a mixture of methanol and dichloromethane (5:95 v/v), to give 4-chloro-6-ethyl-3-formyl-2-methyl-1H-pyrrolo[3,2-c]pyridine as an amorphous solid (6O5 mg); NMR ($d_6$-DMSO): 1.25(t, 3H), 2.8(q, 2H), 7.27(s, 1H), 1O.6(s, 1H), 12-62(br s, 1H).

(ii) Using an analogous procedure to that described in Example 1, part (iv), but starting from 4-chloro-6-ethyl-3-formyl-2-methyl-1H-pyrrolo[3,2-c]pyridine, there was obtained 4-chloro-6-ethyl-3-formyl-2-methyl-1-[(2′-(2-triphenylmethyl-2H-tetrazol-5-yl)biphenyl-4-yl)methyl]-1H-pyrrolo[3,2-c]pyridine as a foam; NMR ($d_6$-DMSO): 1.18(t, 3H), 2.56(s, 3H), 2.7(q, 2H), 5.52(s, 2H), 6.82(m, 6H), 6.9(d, 2H), 7.O7(d, 2H), 7.15-7.68(m, 13H), 7.8(dd, 1H), 1O.7(s, 1H); mass spectrum (+ve FAB, DMSO/NBA): 699 $(M+H)^+$.

## Example 6

Using an analogous procedure to that described in Example 1, but starting from 6-ethyl-3-formyl-2-methyl-1-[(2′-(2-triphenylmethyl-2H-tetrazol-5-yl)biphenyl-4-yl)methyl]-1H-pyrrolo[3,2-c]pyridine (A), there was obtained 6-ethyl-3-formyl-2-methyl-1-[(2′-(1H-tetrazol-5-yl) biphenyl-4-yl)methyl]-1H-pyrrolo[3,2-c]pyridine hydrochloride as a solid after trituration with ether; NMR ($d_6$-DMSO): 1.37(t, 3H), 2.77(s, 3H), 3.O9(q, 2H), 5.74(s, 2H), 7.15-7.38(m, 4H), 7.45-7.7(m, 4H), 8.25(s, 1H), 9.25(s, 1H), 1O.25(s, 1H); mass spectrum (+ve FAB, DMSO/NBA): $(M+H)^+$ 423.

The starting material (A) was obtained as follows:-

(i) A solution of 4-chloro-6-methyl-2-methyl-1H-pyrrolo[3,2-c]pyridine (1 g) in ethanol (15 ml) was catalytically hydrogenated over 1O% palladium on carbon (2OO mg) for 5 hours. The catalyst was removed by filtration and washed with ethanol. The filtrate was concentrated by evaporation to give a solid. The solid was triturated with hot ethyl acetate to give 6-ethyl-2-methyl-1H-pyrrolo[3,2-c]pyridine hydrochloride as a amorphous solid (76O mg); NMR ($d_6$-DMSO): 1.33(t, 3H), 2.49(s, 3H), 3.O4(q, 2H), 6.62(s, 1H), 7.64(s, 1H), 8.92(s, 1H), 12.84(s, 1H); microanalysis, found: C, 59.5; H, 6.6; N, 13.9%; $C_{1O}H_{12}N_2$.HCl.O.25$H_2O$ requires: C, 59.7; H, 6.7; N, 13.9%.

(ii) Using an analogous procedure to that described in Example 5, part (i), but starting from 6-ethyl-2-methyl-1H-pyrrolo[3,2-c]pyridine hydrochloride, there was obtained 6-ethyl-3-formyl-2-methyl-1H-pyrrolo[3,2-c]pyridine as an amorphous solid; NMR ($d_6$-DMSO): 1.25(t, 3H), 2.7(s, 3H), 2.82(q, 2H), 7.21(d, 1H), 9.1O(d, 1H), 1O.O7(s, 1H), 12.1(br s, 1H); mass spectrum (+ve CI): 189 $(M+H)^+$.

(iii) Using an analogous procedure to that described in Example 1, part (iv), but starting from 6-ethyl-3-formyl-2-methyl-1H-pyrrolo[3,2-c]pyridine, there was obtained 6-ethyl-3-formyl-2-methyl-1-[(2′-(2-triphenylmethyl-2H-tetrazol-5-yl)biphenyl-4-yl)methyl]-1H-pyrrolo[3,2-c]pyridine as a gum; NMR ($d_6$-DMSO): 1.2(t, 3H), 2.51(s, 3H), 2.76(q, 2H), 5.49(s, 2H), 6.85(m, 6H), 6.92(d, 2H), 7.O8(d, 2H), 7.2-7.48(m, 11H), 7.58(m, 2H), 7.82(dd, 1H), 9.22(s, 1H), 1O.1(s, 1H); mass spectrum (+ve FAB,

DMSO/NBA/Methanol): 665 $(M+H)^+$.

**Example 7**

Using an analogous procedure to that described in Example 1, but starting from 6-ethyl-4-methoxy-2-methyl-1-[(2′-(2-triphenylmethyl-2H-tetrazol-5-yl)biphenyl-4-yl)methyl]-1H-pyrrolo[3,2-c]pyridine (A), there was obtained 6-ethyl-4-methoxy-2-methyl-1-[(2′-(1H-tetrazol-5-yl) biphenyl-4-yl)methyl]-1H-pyrrolo[3,2-c]pyridine as a solid (after trituration with ether); NMR ($d_6$-DMSO): 1.23(t, 3H), 2.27(s, 3H), 2.67(q, 2H), 3.94(s, 3H), 5.35(s, 2H), 6.25(s, 1H), 6.9O(d, 2H), 6.91(s, 1H), 7.O3(d, 2H), 7.44-7.7(m, 4H); mass spectrum (+ve FAB, DMSO/NBA): 425 $(M+H)^+$; microanalysis, found: C, 69.6; H, 5.7; N, 19.4; $H_2O$, O.8%; $C_{25}H_{24}N_6O.O.25H_2O.O\text{-}16(C_2H_5)_2O$ requires: C, 69.9; H, 6.O; N, 19.1; $H_2O$, 1.O2%.

The starting material (A)-was obtained as follows:-

(i) Trimethyloxonium tetrafluoroborate (84O mg) was added to a stirred solution of 6-ethyl-2-methyl-4,5-dihydro-1H-pyrrolo[3,2-c]pyridin-4-one (1.O g) in dichloromethane (3O ml) under an argon atmosphere. The mixture was stirred for 24 hours. Saturated aqueous sodium hydrogen carbonate solution was added until the aqueous layer was basic (pH8). Dichloromethane (2O ml) was added to the mixture and insoluble solid was removed by filtration. The organic phase was separated and washed with saturated sodium chloride solution and dried ($MgSO_4$). The solvent was removed by evaporation to give a dark brown oil. The oil was purified by flash chromatography eluting with a mixture of methanol and dichloromethane (2:98 v/v) to give 6-ethyl-4-methoxy-2-methyl-1H-pyrrolo[3,2-c]pyridine as a solid (4OO mg); NMR: 1.3O(t, 3H), 2.39(d, 3H), 2.77(q, 2H), 4.O5(s, 3H), 6.22(m, 1H), 6.67(s, 1H), 7.93(br s, 1H); $^{13}$C NMR (CDCl3): ($OCH_3$) 52.84.

(ii) Using an analogous procedure to that described in Example 1, part (iv), but starting from 6-ethyl-4-methoxy-2-methyl-1H-pyrrolo[3,2-c]pyridine, there was obtained 6-ethyl-4-methoxy-2-methyl-1-[(2′-(2-triphenylmethyl-2H-tetrazol-5-yl)biphenyl-4-yl)methyl]-1H-pyrrolo[3,2-c]pyridine as a gum; NMR ($d_6$-DMSO): 1.19(t, 3H), 2.13(d, 3H), 2.6(q, 2H), 3.96(s, 3H), 5.29(s, 2H), 6.26(s, 1H), 6.83(m, 9H), 7.O1(d, 2H), 7.16-7.45(m, 1OH), 7.57(m, 2H), 7.78(dd, 1H); mass spectrum (+ve FAB, DMSO/NBA): 667 $(M+H)^+$.

**Example 8**

Using an analogous procedure to that described in Example 1, but starting from 3-chloro-6-ethyl-2-methyl-1-[(2′-(2-triphenylmethyl-2H- tetrazol-5-yl)biphenyl-4-yl)methyl]-1H-pyrrolo[3,2-c]pyridine (A), there was obtained 3-chloro-6-ethyl-2-methyl-1-[(2′-(1H-tetrazol-5-yl) biphenyl-4-yl)methyl]-1H-pyrrolo-[3,2-c]pyridine hydrochloride as a crystalline solid (after recrystallisation from ethanol), m.p. 198-2O1°C (dec.); NMR ($d_6$-DMSO): 1.35(t, 3H), 2.4(s, 3H), 3.O8(q, 2H), 5.68(s, 2H), 7.O7(m, 4H), 7.4-7.7(m, 4H), 8.18(s, 1H), 9.O6(s, 1H); mass spectrum (+ve FAB, DMSO/NBA): 429 $(M+H)^+$ ; microanalysis, found C, 58.6; H, 5.1; N, 17.2; $H_2O$, 3.9%; $C_{24}H_{21}N_6Cl.HCl.1.25H_2O$ requires: C, 59.1; H, 4.8; N, 17.2; $H_2O$, 4.6%.

The starting material (A) was obtained as follows:-

(i) N-Chlorosuccinimide (15O mg) was added to a stirred solution of 6-ethyl-2-methyl-1Hpyrrolo[3,2-c]pyridine hydrochloride (2OO mg) in dichloromethane (3 ml) and the mixture was stirred for 1.5 hours. Volatile material was removed by evaporation and the residue was triturated with ethyl acetate to give a solid. The solid was collected by filtration and dissolved in dichloromethane (3O ml). The solution was washed with saturated aqueous sodium hydrogen carbonate solution and the organic layer was separated, washed with saturated sodium chloride solution and dried ($MgSO_4$). The solvent was removed by evaporation to give 3-chloro-6-ethyl-2-methyl-1H-pyrrolo[3,2-c]pyridine as a brown amorphous solid; NMR ($d_6$-DMSO): 1.24(t, 3H), 2.35(s, 3H), 2.8(q, 2H), 7.12(s, 1H), 8.54(s, 1H), 11-54(br s, 1H).

(ii) Using an analogous procedure to that described in Example 1, part (iv), but starting from 3-chloro-6-ethyl-2-methyl-1H-pyrrolo[3,2-c]pyridine there was obtained 3-chloro-6-ethyl-2-methyl-1-[(2′-(2-triphenylmethyl-2H-tetrazol-5-yl)biphenyl-4-yl)methyl]-1H-pyrrolo[3,2-c]pyridine as a foam; NMR ($d_6$-DMSO): 1.2O(t, 3H), 2.15(s, 3H), 2.63(q, 2H), 5.39(s, 2H), 6.85(m, 8H), 7.O4(d, 2H), 7.2-7.36(m, 1OH), 7.41(m, 1H), 7.57(m, 2H), 7.8(dd, 1H), 8.64(s, 1H); mass spectrum (+ve FAB, Methanol/NBA): 671 $(M+H)^+$.

**Example 9**

Using an analogous procedure to that described in Example 1, but starting from 4,6-diethyl-2-methoxycarbonyl-1-[(2′-(2-triphenylmethyl-2H-tetrazol-5-yl)biphenyl-4-yl)methyl]-1H-pyrrolo[3,2-c]pyridine (A), there was obtained 4,6-diethyl-2-methoxycarbonyl-1-[(2′-(1H-tetrazol-5-yl)biphenyl-4-yl)methyl]-1H-pyrrolo[3,2-c]pyridine hydrochloride as a crystalline solid (after recrystallisation from methanol/ethyl acetate), m.p. 199-200°C(dec.); NMR (d6-DMSO): 1.36 (m, 6H), 3.07 (q,2H), 3.36 (q,2H), 3.88 (s, 3H), 5.93 (s, 2H), 7.05 (s, 4H), 7.46-7.70 (m, 4H), 8.02 (d, 2H); mass spectrum (+ve FAB, Methanol/NBA): 467 $(M+H)^+$; microanalysis, found: C, 64.1; H, 5.4; N, 16.9%; $C_{27}H_{26}N_6O_2$.HCl requires: C, 64.5; H, 5.4; N, 16.7%.

The starting material (A) was obtained as follows:-

(i) A mixture of methyl 3-aminopentenoate (7.3g) and 5-(1-hydroxypropylidene)-2,2-dimethyl-1,3-dioxane-4,6-dione (23.0g) (obtained as described in J. Org. Chem., 1978, 43, 2087) was heated at 120°C for 2 hours. The mixture was cooled to ambient temperature and triturated with ether (50ml). The product was collected by filtration to give methyl 2,6-diethyl-1,4-dihydro-4-oxopyridine-3-carboxylate (5.2g) as a solid, m.p. 124-127°C; NMR ($d_6$-DMSO), 1.2 (t, 6H), 2.3-2.6 (m, 4H), 3.7 (s, 3H), 5.9 (s, 1H), 11.2 (s, 1H); mass spectrum (chemical ionisation, ammonia): 210 $(M+H)^+$; microanalysis, found: C, 63.1; H, 7.5; N, 6.7%; $C_{11}H_{15}NO_3$ requires: C, 63.1; H, 7.2; N, 6. 7%.

(ii) (4-Methylphenyl)sulphonyl isocyanate (39.12g) was added to a stirred suspension of methyl 2,6-diethyl-1,4-dihydro-4-oxo-pyridine-3-carboxylate (25.0g) in acetonitrile (300ml). After the initial exotherm had subsided the mixture was heated at reflux for 2 hours. The mixture was cooled to ambient temperature and the product was collected by filtration to give methyl 2,6-diethyl-4-(4-methylphenyl)sulphonylaminopyridine-3-carboxylate (37.12g) as a solid, m.p. 185-187°C; NMR ($d_4$-methanol): 1.22 (t,6H), 2.38 (s,3H), 2.62 (two q, 4H), 3.88 (s, 3H), 7.1 (s, 1H), 7.28 (d, 2H), 7.75 (d, 2H); mass spectrum: (chemical ionisation, ammonia): 363 $(M+H)^+$; microanalysis, found: C, 59.3; H, 6.1; N, 7.8%; $C_{18}H_{22}N_2SO_4$ requires 59.6; 6.12; 7.73%.

(iii) A mixture of methyl 2,6-diethyl-4-(4-methylphenyl)sulphonylaminopyridine-3-carboxylate (10g) and lithium aluminium hydride (1.5g) in tetrahydrofuran (300ml) was stirred and heated at reflux for 3 hours. The mixture was then cooled and water (1ml) was added, followed by 2M aqueous hydrochloric acid solution (15ml). The mixture was filtered through diatomaceous earth and the insoluble material washed with ethyl acetate. The combined filtrate was evaporated and the residue obtained was azetroped with toluene (2 x 300ml) to give 2,6-diethyl-3-hydroxymethyl-4-(4-methylphenyl)sulphonylaminopyridine (7.4g) as a crystalline solid (after recrystallisation from methanol/ethyl acetate), m.p. 204-206°C; NMR ($d_6$-DMSO): 1.14 (m, 6H), 2.33 (s, 3H), 2.58 (q, 2H), 2.74 (q, 2H), 4.,47 (s, 2H), 6.98 (s, 1H), 7.27 (d, 2H), 7.70 (d, 2H); mass spectrum (+ve Cl); 335 $(M+H)^+$; microanalysis, found: C, 60.8; H, 6.7; N, 8.3%; $C_{17}H_{22}N_2O_3S$ requires: C, 61.1; H, 6.6; N, 8.4%.

(iv) A mixture of 2,6-diethyl-3-hydroxymethyl-4-(4-methylphenyl)sulphonylaminopyridine (0.334g) and activated manganese dioxide (0.7g) in toluene (20ml) was stirred and heated at reflux for 2 hours. The mixture was then filtered through diatomaceous earth and the filtrate was evaporated to give 2,6-diethyl-3-formyl-4-(4-methylphenyl)sulphonylaminopyridine (0.318g) as a crystalline solid (after recrystallisation from ethyl acetate/hexane), m.p. 158-160C; NMR: 1.27 (m, 6H), 2.41 (s, 3H), 2.74 (q, 2H), 3.07 (q, 2H), 7.27 (s, 1H), 7.3 (d, 2H), 7.85 (d, 2H), 10.32 (s, 1H), 11.6 (br s, 1H); microanalysis, found: C, 60.3; H, 6.2; N, 8.2%; $C_{17}H_{20}N_2O_3S.0.25H_2O$ requires: C, 60.6; H, 6.1; N, 8.3%.

(v) Sodium hydride (60% dispersion in mineral oil; 125mg) was added to a stirred solution of 2,6-diethyl-3-formyl-4-(4-methylphenyl)sulphonylaminopyridine (996mg) in DMF (10ml) cooled in an ice-bath. The mixture was stirred for 30 minutes and then added dropwise to a solution of methylbromoacetate (2.4g) in DMF (5ml) during 15 minutes. The mixture was stirred for 18 hours and then volatile material was removed by evaporation. Water (10ml) was added to the residue and the mixture was extracted with ethyl acetate (2 x 50ml). The combined extracts were washed with saturated sodium chloride solution and dried ($MgSO_4$). The solvent was removed by evaporation to give a gum which was purified by flash chromatography, eluting with a mixture of methanol and dichloromethane (2:98 v/v), to give 2,6-diethyl-3-formyl-4-[N-(4-methylphenyl)sulphonyl-N-methoxycarbonylmethyl]aminopyridine (730mg) as a crystalline solid (after recrystallisation from ethyl acetate/hexane), m.p. 88-90°C; NMR: 1.18 (t, 3H), 1.28 (t, 3H), 2.46 (s, 3H), 2.73 (q, 2H), 3.18 (q, 2H), 3.69 (s, 3H), 4.38 (s, 2H), 6.48 (s, 1H), 7.30 (d, 2H), 7.50 (d, 2H), 10.60 (s, 1H); mass spectrum (+ve Cl): 405 $(M+H)^+$; microanalysis, found: C, 59.9; H, 6.1; N, 7.0%; $C_{20}H_{24}N_2O_5S$ requires: C, 59.4; H, 6.0; N, 6.9%.

(vi) Sodium bis(trimethylsilyl)amide (1M solution in tetrahydrofuran, 3ml) was added dropwise to a solution of 2,6-diethyl-3-formyl-4-[N-(4-methylphenyl)sulphonyl-N-methoxycarbonylmethyl]aminopyridine (1.2g) in tetrahydrofuran (20ml) cooled in an ice-bath and under an atmosphere of argon. The mixture was stirred for 2 hours and then volatile material was removed by evaporation. Water (50ml) was added and the mixture was extracted with chloroform (3 x 30ml). The combined extracts were washed with saturated sodium chloride solution and dried ($MgSO_4$). The solvent was removed by evaporation to give a gum. The gum was dissolved in dichloromethane (20ml) and the solution was cooled in an ice-bath. Pyridine (0.5ml) was added, followed by thionyl chloride (0.25ml). The mixture was stirred for 18 hours and then volatile material was removed by evaporation. Water (50ml) was added to the residue and then saturated sodium hydrogen

carbonate solution was added until the mixture was at pH8. The mixture was extracted with ethyl acetate (3 x 5Oml) and the combined extracts were washed with saturated sodium chloride solution (3Oml) and dried ($MgSO_4$). The solvent was removed by evaporation to give a solid which was purified by flash chromatography, eluting with a mixture of methanol and dichloromethane (5:95v/v), to give methyl 4,6-diethyl-1-[(4-methylphenyl)sulphonyl]-1H-pyrrolo[3,2-c]pyridine-2-carboxylate as a crystalline solid (after recrystallisation from ethyl acetate/hexane), m.p. 129-13O°C; NMR: 1.35 (m,6H), 2.41 (s,3H), 2.98(m,4H), 3.91 (s,3H), 7.25 (s, 1H), 7.32 (d, 2H), 7.75 (s, 1H), 7.97 (d, 2H); mass spectrum (+ve CI): 387 $(M+H)^+$; microanalysis, found: C, 62.6; H, 5.8; N, 7.3%; $C_{20}H_{22}N_2O_4S$ requires: C, 62.2; H, 5.7; N, 7.3%.

(vii) Sodium bis(trimethylsilyl)amide (1M solution in tetrahydrofuran, 3ml) was added to a solution of methyl 4,6-diethyl-1-[(4-methylphenyl)sulphonyl]-1H-pyrrolo[3,2-s]pyridine-2-carboxylate (1.15g) and the mixture was stirred for 3 hours. Volatile material was removed by evaporation and water (2Oml) added to the residue. The mixture was neutralised to pH7 by adding 2M aqueous hydrochloric acid solution and extracted with ethyl acetate (3 x 5Oml). The combined extracts were washed with saturated sodium chloride solution and dried ($MgSO_4$). Solvent was removed by evaporation to give a solid which was purified by flash chromatography, eluting with a mixture of methanol and dichoromethane (5:95 v/v), to give methyl 4,6-diethyl-1H-pyrrolo[3,2-c]pyridine-2-carboxylate as an amorphous solid; NMR ($d_6$-DMSO): 1.26 (m,6H), 2.77 (q,2H), 3.O (q,2H), 3.88 (s,3H), 7.O5 (s,1H), 7.32 (s,1H), 12.1 (br s, 1H); mass spectrum (+ve CI): 233 $(M+H)^+$.

(viii) Using an analogous procedure to that described in Example 1 part (iv), but starting from methyl 4,6-diethyl-1H-pyrrolo-[3,2-c]pyridine-2-carboxylate, there was thus obtained 4,6-diethyl-2-methoxycarbonyl-1-[(2′-(2-triphenylmethyl-2H-tetrazol-5-yl)biphenyl-4-yl)methyl]-1H-pyrrolo[3,2-c]pyridine as a foam; NMR: 1.25 (t,3H), 1.42 (t,3H), 2.8O (q, 2H), 3.11 (q, 2H), 3.81 (s, 3H), 5.66 (s, 2H), 6.8O-6.95 (m, 8H), 7.O3 (d, 2H), 7.17-7.5 (m, 14H), 7.85-7.9O (m, 1H); mass spectrum (+ve FAB, methanol/NBA) 7O9 $(M+H)^+$.

## Example 1O

Using an analogous procedure to that described in Example 1, but starting from 2-cyano-4,6-diethyl-1-[(2′-(2-triphenylmethyl-2H-tetrazol-5-yl)biphenyl-4-yl)methyl]-1H-pyrrolo[3,2-c]pyridine (A), there was obtained 2-cyano-4,6-diethyl-1-[(2′-(1H-tetrazol-5-yl)biphenyl-4-yl)methyl-1H-pyrrolo[3,2-c]pyridine hydrochloride as a crystalline solid (after recrystallisation from ethanol/ethyl acetate), m.p. 246-248°C; NMR ($d_6$-DMSO): 1.36 (t, 3H), 1.4 (t, 3H), 3.1 (q, 2H), 3.37 (q, 2H), 5.75 (s, 2H), 7.12 (d, 2H), 7.19 (d, 2H), 7.48-7.72 (m, 4H), 8.14 (s, 1H), 8.31 (s, 1H); mass spectrum (+ve FAB, methanol/NBA): 434 $(M+H)^+$; microanalysis found: C, 66.1; H, 5.2, N, 21.O%; $C_{26}H_{23}N_7$.HCl requires: C, 66.4; H, 5.2; N, 2O.9%.

The starting material (A) was obtained as follows:-

(i) Using an analogous procedure to that described in Example 9, part (v), but using bromoacetonitrile in place of methylbromoacetate there was obtained 2,6-diethyl-3-formyl-4-[N-(4-methylphenyl)sulphonyl-N-cyanomethyl]aminopyridine as an oil; NMR: 1.21 (t, 3H), 1.3O (t, 3H), 2.47 (s, 3H), 2.78 (q, 2H), 3.15 (q, 2H), 4.61 (s, 2H), 6.7O (s, 1H), 7.33 (d, 2H), 7.58 (d, 2H).

(ii) Sodium bis(trimethylsilyl)amide (1M solution in tetrahydrofuran, 2.2ml) was added to a stirred solution of 2,6-diethyl-3-formyl-4-[N-(4-methylphenyl)sulphonyl-N-cyanomethyl]aminopyridine (O.8g) in DMF (8ml) under an atmosphere of argon. The mixture was stirred for 2.5 hours and the volatile material was removed by evaporation. Water (2Oml) was added to the residue and the mixture was extracted with dichloromethane (7Oml). The extract was washed with saturated sodium chloride solution (15ml) and dried ($MgSO_4$). The solvent was removed by evaporation to give a brown solid which was purified by flash chromatography, eluting with a mixture of methanol and dichloromethane (5:95 v/v), to give 2-cyano-4,6-diethyl-1H-pyrrolo[3,2-c]pyridine as a solid; NMR ($d_6$-DMSO): 1.24 (t, 3H), 1.28 (t, 3H), 2.79 (q, 2H), 2.98 (q, 2H), 7.O7 (s, 1H), 7.56 (d, 1H), 12.5 (br s 1H); mass spectrum (+ve FAB, methanol/NBA): 2OO $(M+H)^+$.

(iii) Using an analogous procedure to that described in Example 1, (part iv), but starting from 2-cyano-4,6-diethyl-1H-pyrrolo[3,2-c] pyridine, there was obtained 2-cyano-4,6-diethyl-1-[(2′-(2-triphenylmethyl-2H-tetrazol-5-yl)biphenyl-4-yl)methyl]-1H-pyrrolo[3,2-c]pyridine as a solid, m.p. 164-166°C (dec); NMR ($d_6$-DMSO): 1.21 (t, 3H), 1.3 (t, 3H), 2.76 (q, 2H), 3.O3 (q, 2H), 5.51 (s, 2H), 6.84 (m, 6H), 7.O6 (s, 4H), 7.18-7.45 (m, 11H), 7.57 (m, 2H), 7.8 (m, 2H); mass spectrum (+ve FAB, $CHCl_3$/NBA): 676 $(M+H)^+$.

## Example 11

Using an analogous procedure to that described in Example 1, but starting from 6-ethyl-2-methyl-4-trifluoromethyl-1[(2′-(2-triphenylmethyl-2H-tetrazol-5-yl)biphenyl-4-yl)methyl]-1H-pyrrolo-[3,2-c]pyridine (A), there was obtained 6-ethyl-2-methyl-4-trifluoromethyl-1-[(2′-(1H-tetrazol-5-yl)biphenyl-4-yl)methyl]-1H-pyrrolo[3,2-c]pyridine as a gum; NMR ($d_6$-DMSO): 1.26(t, 3H), 2.38(s, 3H), 2.84(q, 2H), 5.51(s, 2H), 6.50(s, 1H), 6.95(d, 2H), 7.05(d, 2H), 7.42-7.72(m, 5H); mass spectrum (+ve FAB, Methanol/NBA): 463 $(M+H)^+$.

The starting material (A) was obtained as follows:-

(i) n-Butyllithium (1.6M solution in hexane, 3.2 ml) was added to a solution of 4-chloro-6-ethyl-2-methyl-1H-pyrrolo[3,2-c]pyridine (0.975 g) in tetrahydrofuran (10 ml) cooled to -78°C under an atmosphere of argon. The mixture was stirred and allowed to reach 0°C during 1 hour. The mixture was then re-cooled to -78°C and benzenesulphonyl chloride (0.7 ml) was added. The mixture was allowed to reach ambient temperature and stirred for 18 hours. Water (10 ml) was added and the mixture was extracted with ethyl acetate (2 x 10 ml). The combined extracts were washed successively with saturated sodium carbonate solution (5 ml), water (5 ml) and saturated sodium chloride solution (5 ml). The organic phase was dried ($MgSO_4$) and the solvent was removed by evaporation to give a solid. The solid was purified by flash chromatography, eluting with a mixture of methanol and dichloromethane (2:98 v/v), to give 4-chloro-6-ethyl-2-methyl-1-phenylsulphonyl-1H-pyrrolo[3,2-c]pyridine as a solid (1.04 gm), m.p. 131-135°C; NMR: 1.35(t, 3H), 2.57(s, 3H), 2.90(q, 2H), 6.42(s, 1H), 7.43-7.68(m, 3H), 7.74-7.90(m, 3H); mass spectrum (+ve CI): 335 $(M+H)^+$.

(ii) A mixture of 4-chloro-6-ethyl-2-methyl-1-phenylsulphonyl-1H-pyrrolo[3,2-c]pyridine (0.67 g), sodium iodide (0.9 g), hydriodic acid (66% aqueous solution, 0.2 ml) in methyl ethyl ketone (10 ml) was stirred and refluxed for 24 hours. Water (20 ml) was added and the mixture was extracted with ethyl acetate (2 x 50 ml). The combined extracts were washed with saturated sodium chloride solution and dried ($MgSO_4$). The solvent was removed by evaporation to give a solid which was purified by flash chromatography, eluting with a mixture of methanol and dichloromethane (2:98 v/v), to give 6-ethyl-4-iodo-2-methyl-1-phenylsulphonyl-1H-pyrrolo[3,2-c]pyridine (0.6 g) as an amorphous solid, m.p. 125-128°C; mass spectrum (+ve CI): 427 $(M+H)^+$.

(iii) A mixture of 6-ethyl-4-iodo-2-methyl-1-phenylsulphonyl-1H-pyrrolo[3,2-c]pyridine (580 mg), copper (I) iodide (428 mg), potassium fluoride (104 mg) and triethyl(trifluoromethyl)silane (0.3 ml) in DMF (2 ml) and N-methylpyrrolidone (1.5 ml) was stirred and heated at 80°C for 24 hours in a screw-capped reaction tube. The mixture was cooled, poured into water (50 ml) and extracted with ethyl acetate (3 x 20 ml). The combined extracts were washed with water (5 ml) and dried ($MgSO_4$). The solvent was evaporated to give a gum (392 mg) which was purified by flash chromatography, eluting with a mixture of methanol and dichloromethane (1:99 v/v), to give 6-ethyl-2-methyl-1-phenylsulphonyl-4-trifluoromethyl-1H-pyrrolo[3,2-c]pyridine (210 mg); NMR ($d_6$-DMSO): 1.29(t, 3H), 2.64(d, 3H), 2.95(q, 2H), 6.78(m, 1H), 7.59-7.87(m, 3H), 7.94-8.05(m, 2H), 8.13(s, 1H); mass spectrum (+ve FAB, Methanol/NBA): 369 $(M+H)^+$.

(iv) A mixture of 6-ethyl-2-methyl-1-phenylsulphonyl-4-trifluoromethyl-1H-pyrrolo[3,2-c]pyridine (200 mg) and 2M aqueous sodium hydroxide solution (1 ml) in methanol (5 ml) was heated at reflux for 2 hours. Volatile material was removed by evaporation. Water (2 ml) added to the residue and the mixture was extracted with ethyl acetate (2 x 5 ml). The combined extracts were washed with saturated sodium chloride solution (2 ml) and dried ($MgSO_4$). The solvent was removed by evaporation to give a solid (95 mg). Sodium hydride (60% dispersion in mineral oil, 20 mg) was added to a solution of the solid in DMF (3 ml). The mixture was stirred until evolution of hydrogen had ceased and then 5-[2-(41-bromomethylbiphenyl)]triphenylmethyl-2H-tetrazole (300 mg) was added. The mixture was stirred for 18 hours. Water (40 ml) was then added and the mixture was extracted with ethyl acetate (2 x 10 ml). The combined extracts were washed with saturated sodium chloride solution (5 ml) and dried ($MgSO_4$) . The solvent was removed by evaporation to give a gum which was purified by flash chromatography, eluting with dichloromethane, to give 6-ethyl-2-methyl-4-trifluoromethyl-1-[(2′-(2-triphenylmethyl-2H-tetrazol-5-yl)biphenyl-4-yl)methyl]-1H-pyrrolo[3,2-c]pyridine as a solid (165 mg), m.p. 191-193°C (dec.); NMR: 1.25(t, 3H), 2.24(s, 3H), 2.83(q, 2H), 5.19(s, 2H), 6.53(s, 1H), 6.70(d, 2H), 6.88-6.95(m, 6H), 7.08(d, 2H), 7.15-7.38(m, 11H), 7.42-7.50(m, 2H), 7.89-7.95(m, 1H); mass spectrum (+ve FAB, $CHCl_3$/NBA): 705 $(M+H)^+$.

## Example 12

(Note: all parts by weight)

The compounds of the invention may be administered for therapeutic or prophylactic use to warm-blooded animals such as man in the form of conventional pharmaceutical compositions, typical examples of which include the following:-

a) **Capsule** (for oral administration)

| | |
|---|---|
| Active ingredient * | 20 |

| | |
|---|---|
| Lactose powder | 578.5 |
| Magnesium stearate | 1.5 |

b) **Tablet** (for oral administration)

| | |
|---|---|
| Active ingredient * | 50 |
| Microcrystalline cellulose | 400 |
| Starch (pregelatinised) | 47.5 |
| Magnesium stearate | 2.5 |

c) **Injectable Solution** (for intravenous administration)

| | |
|---|---|
| Active ingredient * | 0.05 - 1.0 |
| Propylene glycol | 5.0 |
| Polyethylene glycol (300) | 3.0 - 5.0 |
| Purified water | to 100% |

d) **Injectable Suspension** (for intramuscular administration

| | |
|---|---|
| Active ingredient * | 0.05 - 1.0 |
| Methylcellulose | 0.5 |
| Tween 80 | 0.05 |
| Benzyl alcohol | 0.9 |
| Benzalkonium chloride | 0.1 |
| Purified water | to 100% |

Note: the active ingredient * may typically be an Example described hereinbefore and will conveniently be present as a pharmaceutically acceptable acid-addition salt, such as the hydrochloride salt. Tablets and capsules formulations may be coated in conventional manner in order to modify or sustain dissolution of the active ingredient. Thus, for example, they may be coated with a conventional enterically digestible coating.

**Chemical Formulae**

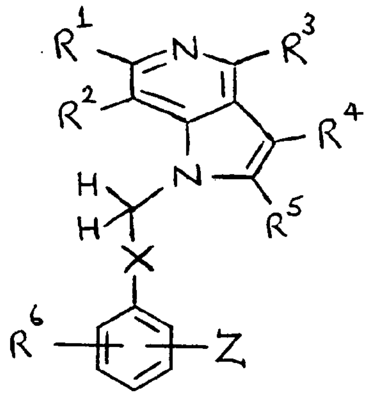

I

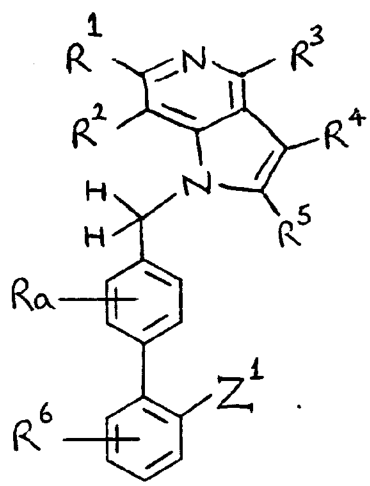

Ia

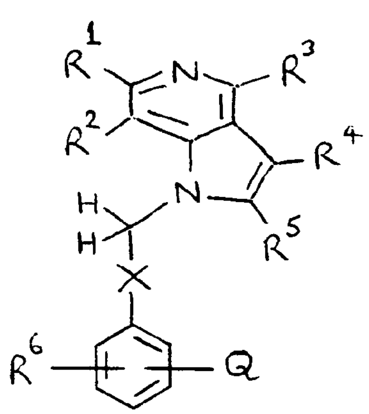

II

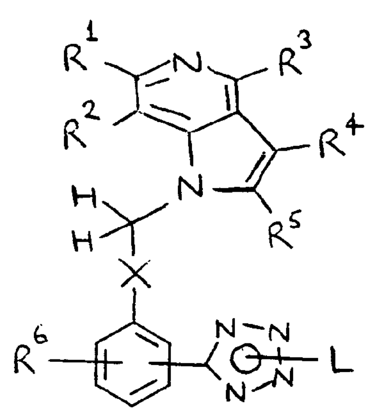

III

**Chemical Formulae**

**(continued)**

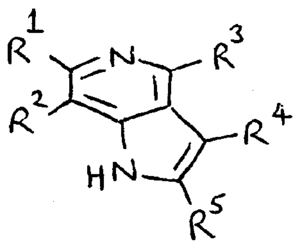

IV

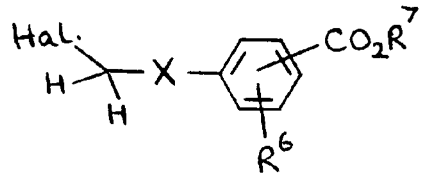

V

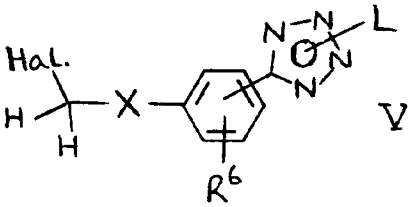

VI

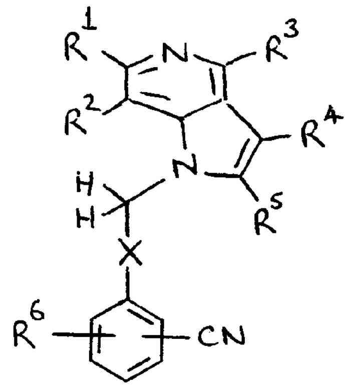

VII

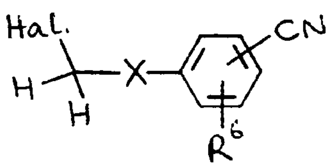

VIII

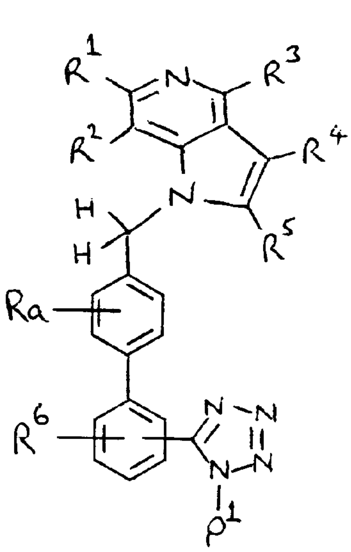

IX

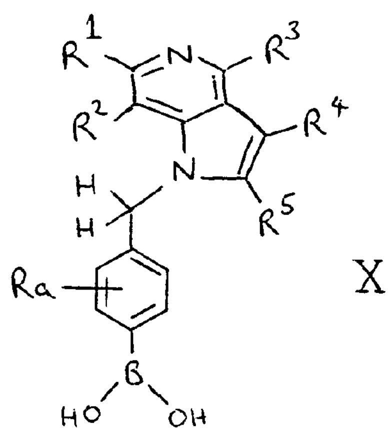

X

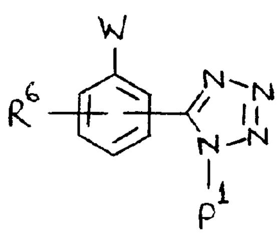

XI

Scheme 1

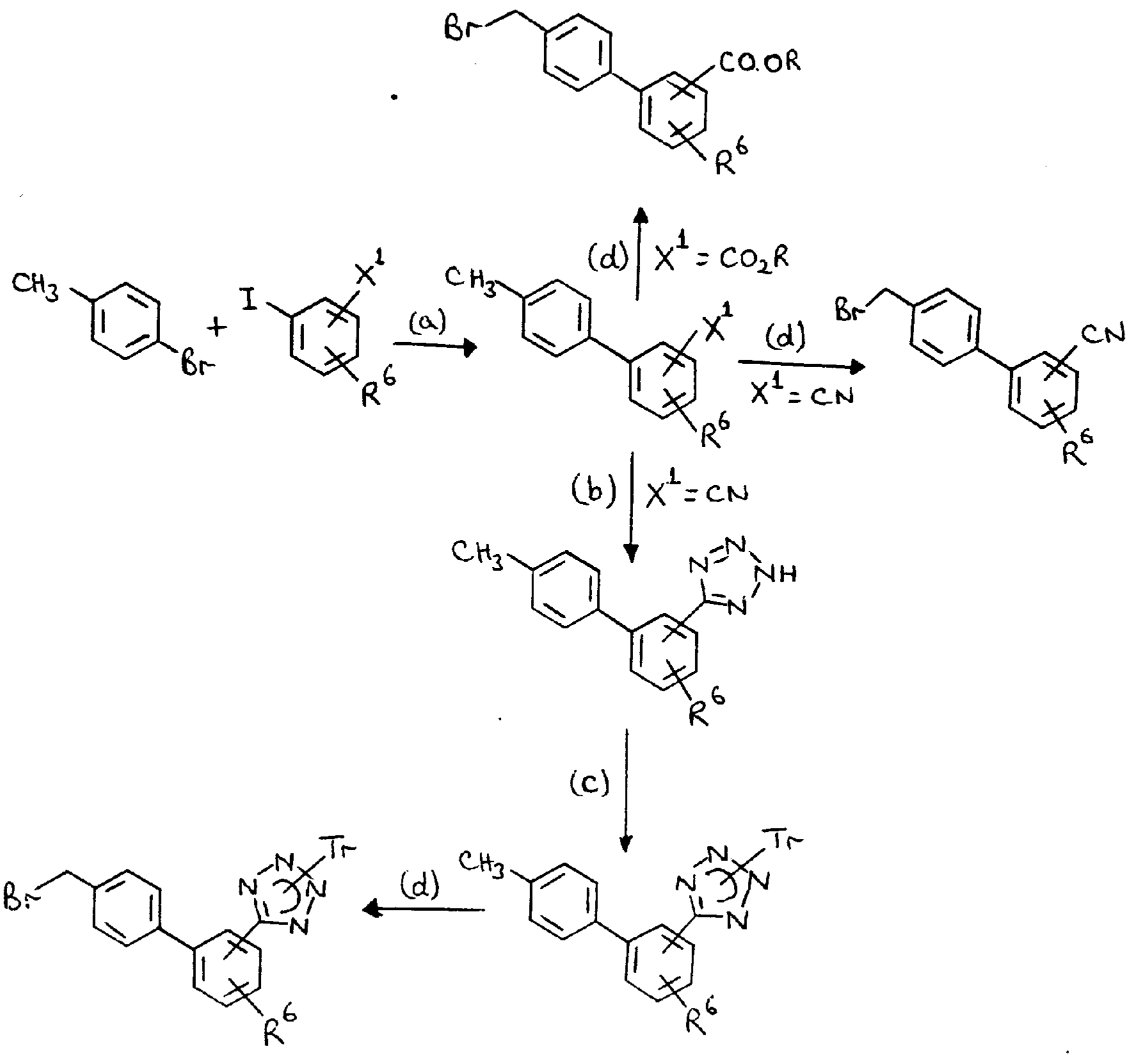


Note: R = lower alkyl, benzyl, phenyl; Tr = triphenylmethyl (trityl)

Reagents: a) BuLi/THF; $ZnCl_2/Et_2O$; $Pd(Ph_3P)_4$
b) $Bu_3Sn.N_3$/toluene; HCl/toluene
c) $Tr.Cl/Et_3N/CH_2Cl_2$
d) N-bromosuccinimide/azoisobutyronitrile/$CCl_4$

Scheme 2

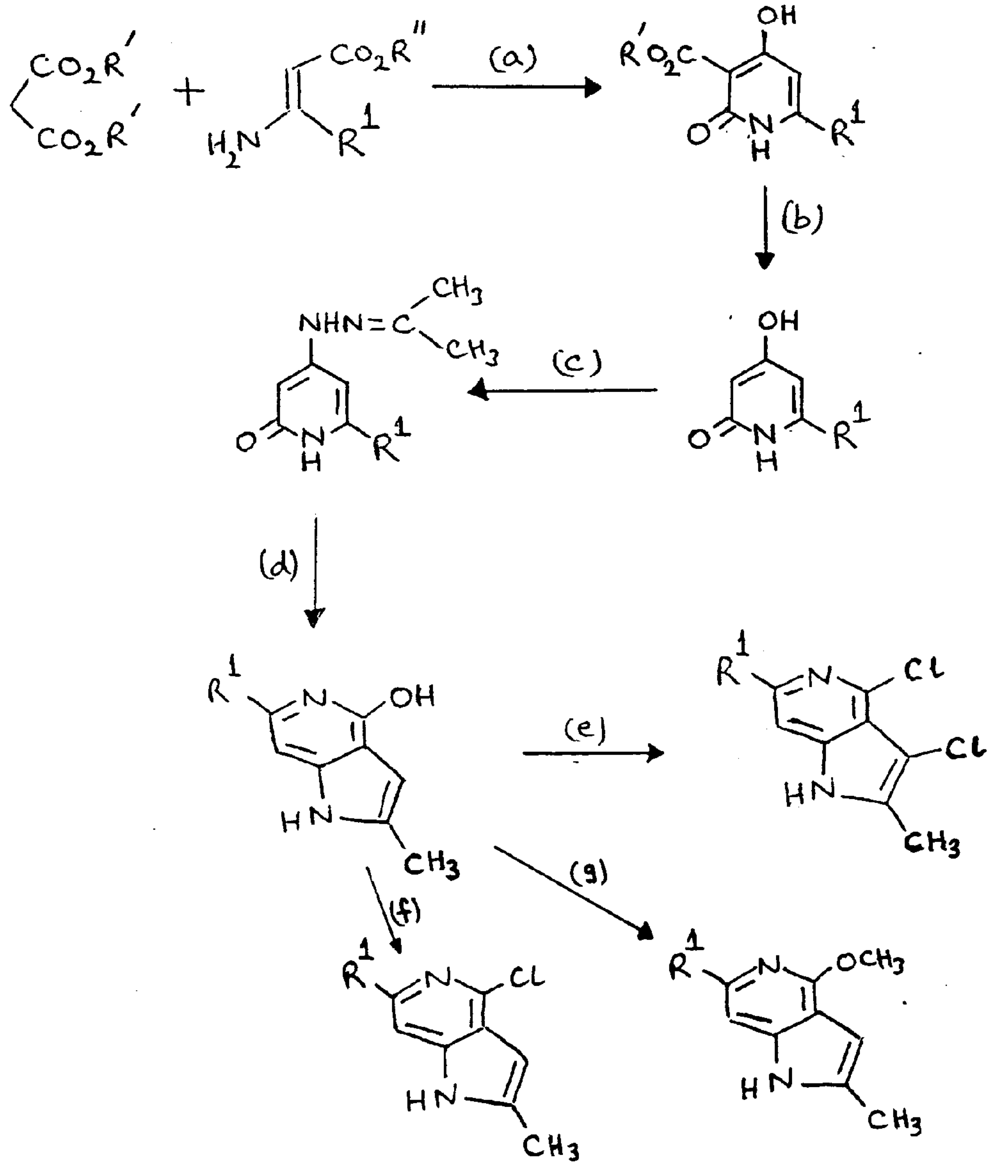


Note: R′ and R″ = lower alkyl;

Reagents: (a) NaOMe, heat
(b) (i) NaOH (ii) HCl (iii) heat
(c) hydrazine hydrate, 2-ethoxyethanol, reflux; then acetone, reflux
(d) Ph-Ph/Ph-O-Ph mixture, reflux
(e) $POCl_3$, $PCl_5$, heat
(f) $POCl_3$ (freshly distilled), reflux
(g) $(CH_3)_3OBF_4$, dichloromethane

Scheme 3

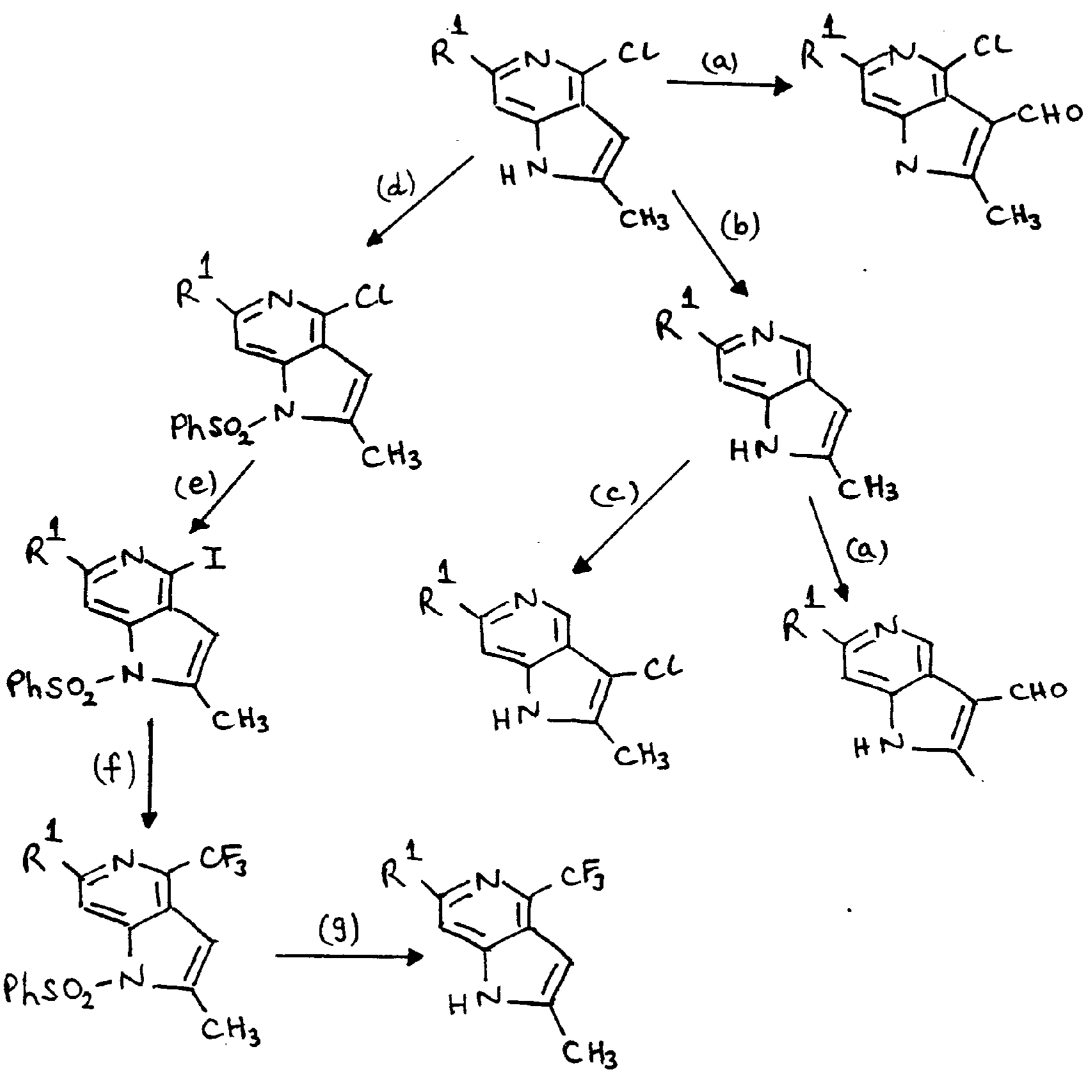


------------------------------------------------------------

Note: Ph = phenyl

Reagents: (a) $POCl_3$/DMF
(b) Catalytic hydrogenation over palladium on carbon
(c) N-Chlorosuccinimide, dichloromethane
(d) (i) n-BuLi, THF/hexane, -78 to 0°C
(ii) $PhSO_2Cl$, THF, -78 to ambient
(e) NaI, aqueous HI, methyl ethyl ketone, reflux
(f) CuI, KF, triethyl(trifluoromethyl)silane, DMF/NMP, 80°C
(g) NaOH, aqu. methanol, reflux

Scheme 4

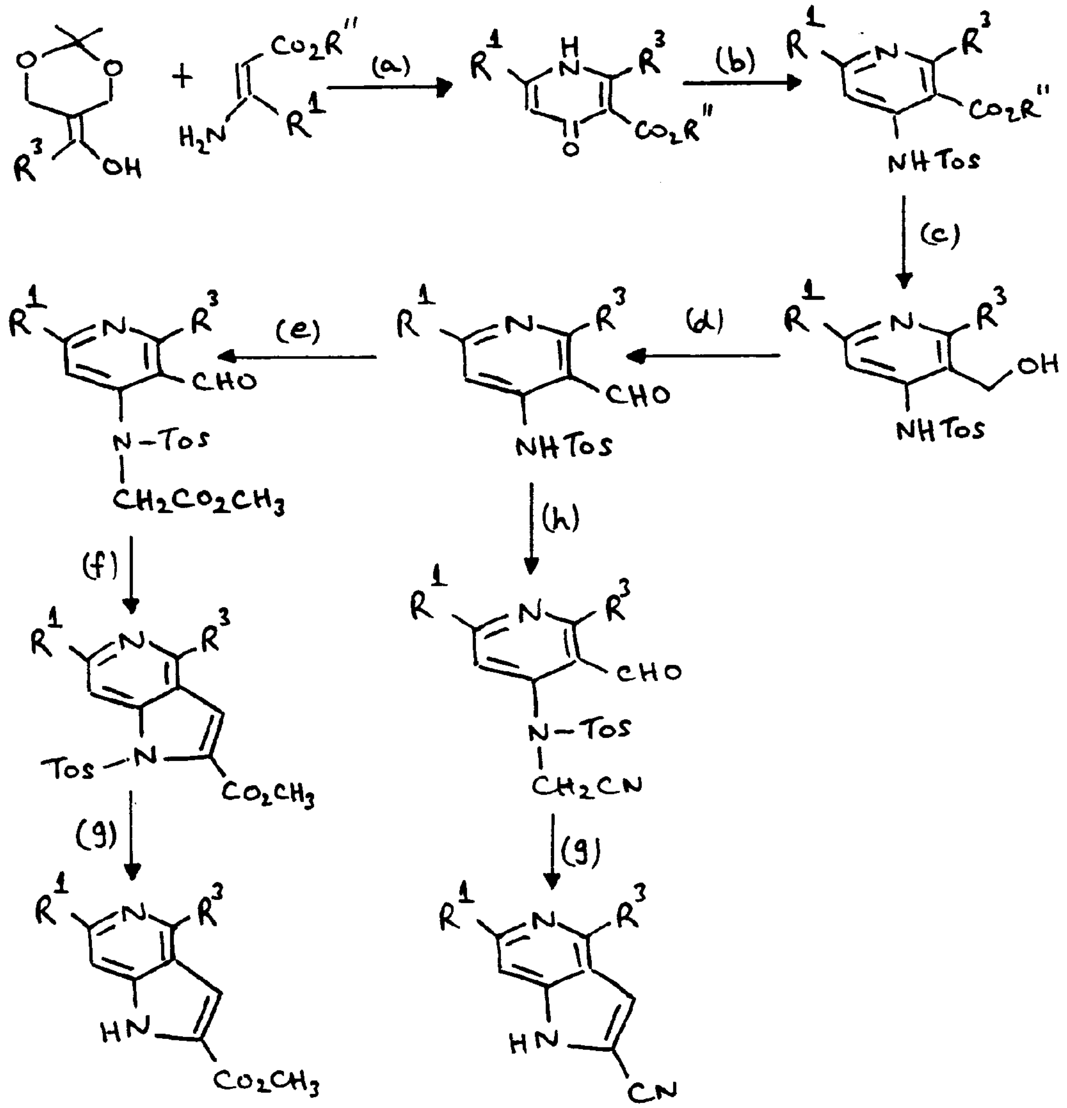


Note: R" = lower alkyl; Tos = p-toluenesulphonyl (tosyl)

Reagents:
(a) Heat, 120°C
(b) p-Toluenesulphonyl isocyanate, acetonitrile, reflux
(c) Lithium aluminium hydride, THF, reflux
(d) Activated $MnO_2$, toluene, reflux
(e) NaH, DMF, $BrCH_2CO_2CH_3$, 0°C
(f) (i) $NaN(SiMe_3)_2$, THF, 0°C; (ii) Pyridine, $SOCl_2$, 0°C
(g) $NaN(SiMe_3)_2$, THF
(h) NaH, DMF, $BrCH_2CN$, 0°C

Scheme 5

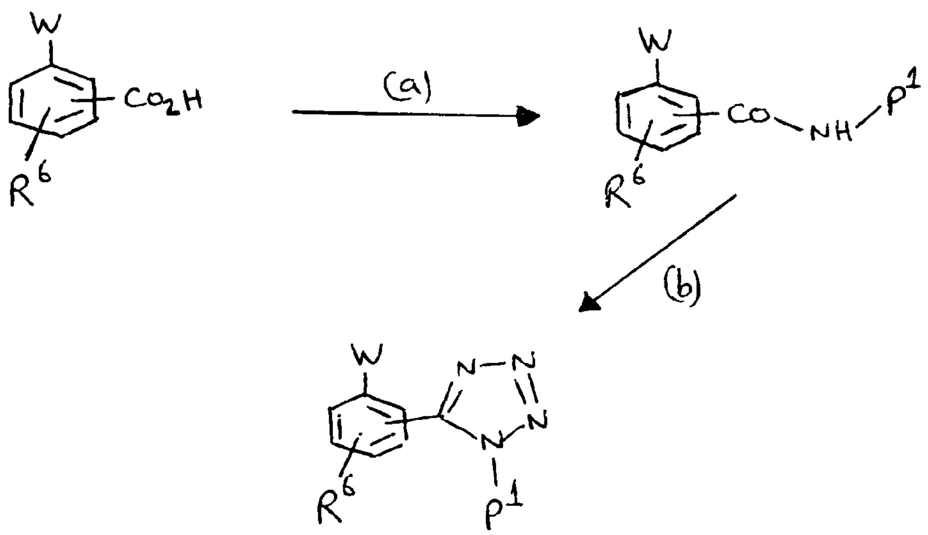


---

Reagents: (a) thionyl chloride, DMF, toluene, 80°C; then add to $P^1.NH_2$, toluene, NMP, ambient temperature

(b) (i) triethylamine, acetonitrile, DMF;

(ii) thionyl chloride, 10°C; and

(iii) triethylamine, sodium azide, tetrabutylammonium bromide, 10°C to ambient temperature

## Claims

1. A pyrrolopyridine derivative of the formula I

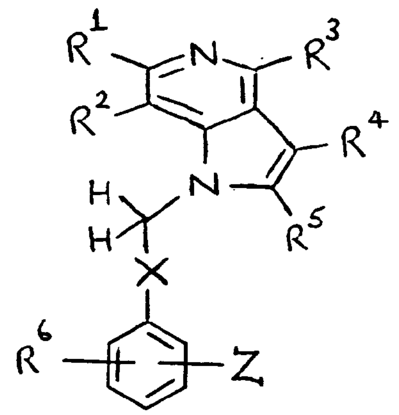

I

wherein $R^1$ is hydrogen, (1-8C)alkyl, (3-8C)cycloalkyl, phenyl or substituted (1-4C)alkyl, the latter containing one or more fluoro substituents or bearing a (3-8C)cycloalkyl, (1-4C)alkoxy or phenyl substituent; $R^2$ is hydrogen, (1-4C)alkyl, (1-4C)alkoxy, halogeno, trifluoromethyl, carboxy, (1-4C)alkoxycarbonyl, (3-6C)alkenyloxycarbonyl, cyano, nitro, carbamoyl, (1-4C)alkanoyl, N-alkylcarbamoyl and di-(N-alkyl)carbamoyl of up to 7 carbon atoms, amino, alkylamino and dialkylamino of up to 6 carbon atoms, 3-(1-4C)alkylureido and (1-4C)alkanoylamino; $R^3$ is selected from halogeno, (1-4C)alkoxy, hydroxy, amino, alkylamino and dialkylamino of up to 6 carbon atoms, and any of the values defined for $R^1$; $R^4$ is hydrogen, (1-4C)alkyl, (1-4C)alkyl containing one or more fluoro substituents, (1-4C)alkoxy, halogeno, carboxy, (1-4C)alkoxycarbonyl, (3-6C)alkenyloxycarbonyl, cyano, nitro, (1-4C)alkanoyl, (1-4C)alkylthio, (1-4C)alkylsulphinyl, (1-4C)alkylsulphonyl or phenylsulphonyl; $R^5$ is hydrogen, (1-8C)alkyl, (1-4C)alkyl containing one or more fluoro substituents, hydroxy(1-4C)alkyl, carboxy, (1-4C)alkoxycarbonyl, (3-6C)alkenyloxycarbonyl, cyano, (1-4C)alkanoyl, (1-4C)alkylthio, (1-4C)alkylsulphinyl, (1-4C)alkylsulphonyl, phenylsulphonyl, (3-8C)cycloalkyl, (3-8C)cycloalkyl(1-4C)alkyl, phenyl or phenyl(1-4C)alkyl; $R^6$ is selected from hydrogen, (1-4C)alkyl, (1-4C)alkoxy, halogeno, trifluoromethyl, cyano and nitro; X is phenylene optionally bearing a substituent selected from (1-4C)alkyl, (1-4C)alkoxy, halogeno, (1-4C)alkanoyl, trifluoromethyl, cyano and nitro, or X is a direct bond between the adjacent phenyl and methylene groups; Z is 1H-tetrazol-5-yl, -CO.NH.(1H-tetrazol-5-yl) or a group of the formula **-CO.OR$^7$** or **-CO.NH.SO$_2$.R$^8$** in which $R^7$ is hydrogen or a non-toxic, biodegradable residue of a physiologically acceptable alcohol or phenol, and $R^8$ is (1-6C)alkyl, (3-8C)cycloalkyl or phenyl; and wherein any of said phenyl moieties may be unsubstituted or bear one or two substituents independently selected from (1-4C)alkyl, (1-4C)alkoxy, halogeno, cyano and trifluoromethyl; or an N-oxide thereof; or a non-toxic salt thereof.

2. A compound as claimed in claim 1 wherein $R^1$ is hydrogen, methyl, ethyl, propyl, isopropyl, butyl, isobutyl, sec-butyl, pentyl, hexyl, cyclopropyl, cyclopentyl, cyclohexyl, phenyl, fluoromethyl, trifluoromethyl, 2,2,2-trifluoroethyl, pentafluoroethyl, cyclopropylmethyl, cyclopentylmethyl, cyclohexylmethyl, 2-methoxyethyl, 2-ethoxyethyl, benzyl, 1-phenylethyl or 2-phenylethyl; $R^2$ is methyl, ethyl, propyl, isopropyl, butyl, methoxy, ethoxy, fluoro, chloro, bromo, iodo, trifluoromethyl, carboxy, methoxycarbonyl, ethoxycarbonyl, propoxycarbonyl, allyloxycarbonyl, 2-methyl-2-propenyloxycarbonyl, 3-methyl-3-butenyloxycarbonyl, cyano, nitro, carbamoyl, formyl, acetyl, propionyl, N-methylcarbamoyl, N-ethylcarbamoyl, N,N-dimethylcarbamoyl, N,N-diethylcarbamoyl, amino, methylamino, ethylamino, butylamino, dimethylamino, diethylamino, dipropylamino, 3-methylureido, 3-ethylureido, 3-propylureido, formamido, acetamido or propanamido; $R^3$ is selected from hydrogen, methyl, ethyl, propyl, isopropyl, butyl, isobutyl, sec-butyl, pentyl, hexyl, cyclopropyl, cyclopentyl, cyclohexyl, phenyl, fluoromethyl, trifluoromethyl, 2,2,2-trifluoroethyl, pentafluoroethyl, cyclopropylmethyl, cyclopentylmethyl, cyclohexylmethyl, 2-methoxyethyl, 2-ethoxyethyl, benzyl, 1-phenylethyl, 2-phenylethyl, methoxy, ethoxy, fluoro, chloro, bromo, iodo, hydroxy, amino, methylamino, ethylamino, butylamino, dimethylamino and diethylamino; $R^4$ is hydrogen, methyl, ethyl, propyl, isopropyl, butyl, fluoromethyl, trifluoromethyl, 2,2,2-trifluoroethyl, pentafluoroethyl, methoxy, ethoxy, fluoro, chloro, bromo, iodo, carboxy, methoxycarbonyl, ethoxycarbonyl, propoxycarbonyl, allyloxycarbonyl, 2-methyl-2-propenyloxycarbonyl, 3-methyl-3-butenyloxycarbonyl, cyano, nitro, formyl, acetyl, propionyl, methythio, ethylthio, methylsulphinyl, ethylsulphinyl, methylsulphonyl, ethylsulphonyl or phenylsulphonyl; $R^5$ is hydrogen, methyl, ethyl, propyl, isopropyl, butyl, isobutyl, sec-butyl, pentyl, hexyl, fluoromethyl, trifluoromethyl, 2,2,2-trifluoroethyl, pentafluoroethyl, hydroxymethyl, 1-hydroxyethyl, 2-hydroxyethyl, carboxy, methoxycarbonyl, ethoxycarbonyl, propoxycarbonyl, allyloxycarbonyl, cyano, formyl, acetyl, propionyl, methythio, ethylthio, methylsulphinyl, ethylsulphinyl, methylsulphonyl, ethylsulphonyl or phenylsulphonyl, 2-methyl-2-propenyloxycarbonyl, 3-methyl-3-butenyloxycarbonyl, cyclopropyl, cyclopentyl, cyclohexyl, cyclopropylmethyl, cyclopentylmethyl, cyclohexylmethyl, 2-cyclopentylethyl, phenyl, benzyl, 1-phenylethyl or 2-phenylethyl; $R^6$ is hydrogen, methyl, ethyl, propyl, isopropyl, butyl, methoxy, ethoxy, fluoro, chloro, bromo, iodo, trifluoromethyl, cyano or nitro; X is phenylene optionally bearing a substituent selected from methyl, ethyl, methoxy, ethoxy, fluoro, chloro, bromo, iodo, formyl, acetyl, propionyl, trifluoromethyl, cyano or nitro, or X is a direct bond between the adjacent phenyl and methylene groups; $R^7$ is hydrogen or a residue derived from a (1-6C)alkanol, or phenol or glycerol; and $R^8$ is methyl, ethyl, propyl, isopropyl, butyl, pentyl, cyclobutyl, cyclopentyl, cyclohexyl or phenyl; and wherein any of said phenyl moieties may be unsubstituted or bear one or two substituents selected from methyl, ethyl, methoxy, ethoxy, fluoro, chloro, bromo, cyano and trifluoromethyl.

3. A compound as claimed in claim 1 or 2 wherein $R^5$ is hydrogen, (1-8C)alkyl, hydroxy(1-4C)alkyl, carboxy, (1-4C)alkoxycarbonyl, (3-6C)alkenyloxycarbonyl, (3-8C)cycloalkyl, (3-8C)cycloalkyl(1-4C)alkyl, phenyl or phenyl(1-4C)alkyl, and wherein the phenyl rings of the latter two groups may optionally bear one or two substituents independently selected from (1-4C)alkyl, (1-4C)alkoxy, halogeno, cyano and trifluoromethyl.

4. A compound of the formula Ia

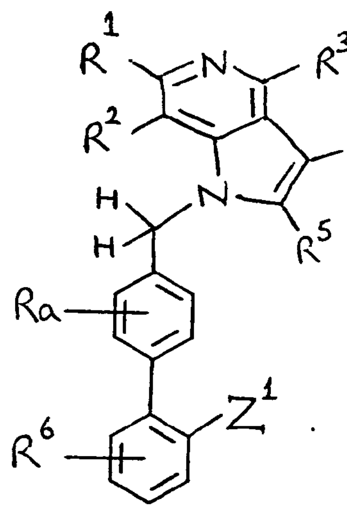

Ia

wherein $R^1$, $R^2$, $R^3$, $R^4$, $R^5$ and $R^6$ have any of the meanings defined in claim 1, 2 or 3; Ra is selected from hydrogen, (1-4C)alkyl, (1-4C)alkoxy, halogeno, (1-4C)alkanoyl, trifluoromethyl, cyano and nitro; and $Z^1$ is carboxy or 1H-tetrazol-5-yl; and the non-toxic salts thereof.

5. A compound as claimed in claim 4 wherein $R^1$ is (1-4C)alkyl; $R^2$ is hydrogen; $R^3$ is hydrogen, (1-4C)alkyl, (1-4C)alkyl containing one or more fluoro substituents, (1-4C)alkoxy or halogeno; $R^4$ is hydrogen, halogeno, or formyl; $R^5$ is (1-4C)alkyl, (1-4C)alkoxycarbonyl or cyano; $R^6$ and Ra are both hydrogen; and $Z^1$ is 1H-tetrazol-5-yl.

6. A compound of the formula I selected from :-
3,4-dichloro-6-ethyl-2-methyl-1-[(2′-(1H-tetrazol-5-yl)biphenyl-4-yl)methyl]-1H-pyrrolo[3,2-c]pyridine;
4-chloro-2,6-dimethyl-1-[(2′-(1H-tetrazol-5-yl)biphenyl-4-yl)methyl]-1H-pyrrolo[3,2-c]pyridine;
4-chloro-6-ethyl-2-methyl-1-[(2′-(1H-tetrazol-5-yl)biphenyl-4-yl)methyl]-1H-pyrrolo[3,2-c]pyridine;
4-chloro-6-ethyl-3-formyl-2-methyl-1-[(2′-(1H-tetrazol-5-yl)biphenyl-4-yl)methyl]-1H-pyrrolo[3,2-c]pyridine;
6-ethyl-3-formyl-2-methyl-1-[(2′-(1H-tetrazol-5-yl)biphenyl-4-yl)methyl]-1H-pyrrolo[3,2-c]pyridine;
3-chloro-6-ethyl-2-methyl-1-[(2′-(1H-tetrazol-5-yl)biphenyl-4-yl)methyl]-1H-pyrrolo-[3,2-c]pyridine; and

the non-toxic salts thereof.

7. A salt as claimed in any one preceding claim which is selected from salts with acids forming physiologically acceptable anions and, for those compounds of formula I which are acidic, alkali metal, alkaline earth metal, aluminium and ammonium salts, and salts with organic bases affording physiologically acceptable cations.

8. A process for the manufacture of a compound of formula I or a non-toxic salt thereof, as claimed in claim 1, which is characterised in that;-

(a) For those compounds of formula I in which Z is carboxy, a carboxylic acid derivative of the formula II

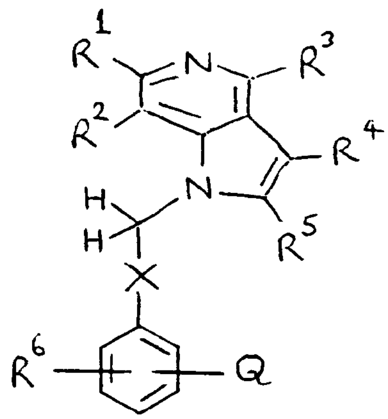

II

in which Q is a protected carboxy group selected from (1-6C)alkoxycarbonyl, phenoxycarbonyl, benzyloxycarbonyl and carbamoyl, is converted to carboxy;

(b) For those compounds of formula I wherein Z is tetrazolyl, a compound of formula III

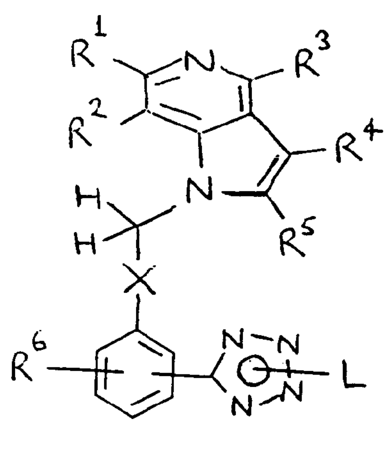

III

in which L is a suitable protecting group affixed to a nitrogen of the tetrazolyl moiety, is deprotected;

(c) A compound of the formula IV

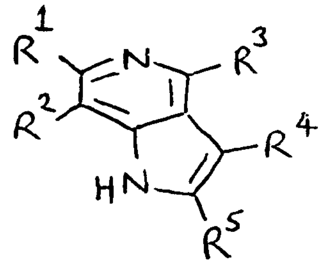

IV

is alkylated with a compound of the formula V

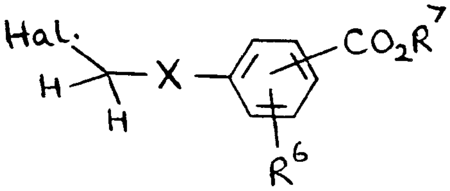

V

wherein **Hal.** stands for a suitable leaving group; or

(d) For a compound of the formula I wherein Z is tetrazolyl, X is p-phenylene optionally bearing a substituent selected from (1-4C)alkyl, (1-4C)alkoxy, halogeno, trifluoromethyl, cyano and nitro, a compound of the formula IX

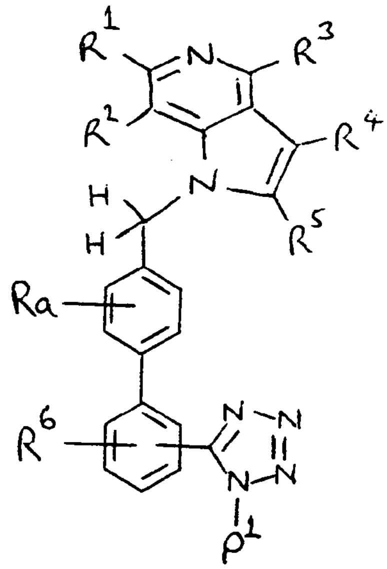

IX

wherein $P^1$ is an electron-deficient phenyl group; Ra is hydrogen, (1-4C)alkyl, (1-4C)alkoxy, halogeno, trifluoromethyl, cyano or nitro, is reacted with a base selected from an alkali metal hydroxide, (1-12C)alkanolate, (1-12C)alkanethiolate, phenolate, thiophenolate or diphenylphosphide, wherein any phenyl ring of the latter three groups may optionally bear a (1-4C)alkyl, (1-4C)alkoxy or halogeno group;

whereafter: when a compound of the formula I is required wherein Z is 1H-tetrazol-5-yl, a compound of the formula I wherein Z is a group of the formula -CO.OR$^7$ is converted into the corresponding nitrile under standard conditions, followed by reaction of the nitrile with an azide;

when a compound of the formula I is required wherein $R^3$ is hydrogen, a compound of the formula I wherein $R^3$ is halogeno is catalytically hydrogenated;

when a compound of the formula I is required wherein Z is a group of the formula **-CO.NH.SO$_2$R$^8$** or a group of the formula **-CO.OR$^7$** in which $R^7$ is other than hydrogen, a carboxylic acid of the formula I in which Z is carboxy (or a reactive derivative of said acid) is reacted with a sulphonamide of the formula **NH$_2$.SO$_2$R$^8$** or a hydroxy compound of the formula **HO.R$^7$**, or with a salt thereof;

when an N-oxide of a compound of the formula I is required, a compound of the formula I is oxidised;

when a non-toxic salt of a compound of formula I is required, it is obtained by reaction with the appropriate acid or base affording a physiologically acceptable ion, or by any other conventional salt formation procedure; and

when an optically active form of a compound of formula I is required, one of the aforesaid processes (a)-(d) is carried out using an optically active starting material, or the racemic form of a compound of formula I in which Z is an acidic group is resolved by reaction with an optically active form of a suitable organic base followed by conventional separation of the diastereoisomeric mixture of salts thus obtained, and liberation of the required optically active form of said compound of formula I by conventional treatment with acid;

and wherein $R^1$, $R^2$, $R^3$, $R^4$, $R^5$, $R^6$, $R^7$, $R^8$, Ra, X and Z have any of the meanings defined in any of claims 1 to 5 unless otherwise stated.

**9.** A pharmaceutical composition which comprises a compound of the formula I or Ia, or a non-toxic salt thereof, as claimed in any of claims 1 to 7, together with a pharmaceutically acceptable diluent or carrier.

**10.** A compound of the formula III wherein $R^1$, $R^2$, $R^3$, $R^4$, $R^5$, $R^6$ and X have any of the meanings defined in any of claims 1 to 5, and L is a protecting group.

**Claim for the following Contracting State : GR**

**1.** A process for the manufacture of a pyrrolopyridine derivative of the formula I

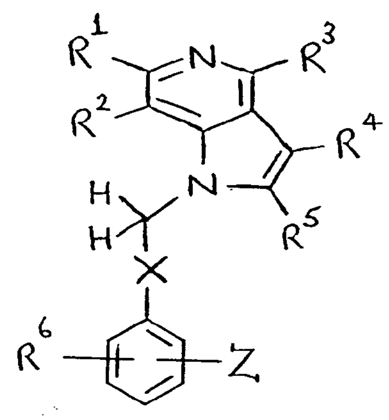

I

wherein $R^1$ is hydrogen, (1-8C)alkyl, (3-8C)-cycloalkyl, phenyl or substituted (1-4C)alkyl, the latter containing one or more fluoro substituents or bearing a (3-8C)cycloalkyl, (1-4C)alkoxy or phenyl substituent; $R^2$ is hydrogen, (1-4C)alkyl, (1-4C)alkoxy, halogeno, trifluoromethyl, carboxy, (1-4C)alkoxycarbonyl, (3-6C)alkenyloxycarbonyl, cyano, nitro, carbamoyl, (1-4C)alkanoyl, N-alkylcarbamoyl and di-(N-alkyl)carbamoyl of up to 7 carbon atoms, amino, alkylamino and dialkylamino of up to 6 carbon atoms, 3-(1-4C)alkylureido and (1-4C)alkanoylamino; $R^3$ is selected from halogeno, (1-4C)alkoxy, hydroxy, amino, alkylamino and dialkylamino of up to 6 carbon atoms, and any of the values defined for $R^1$; $R^4$ is hydrogen, (1-4C)alkyl, (1-4C)alkyl containing one or more fluoro substituents, (1-4C)alkoxy, halogeno, carboxy, (1-4C)alkoxycarbonyl, (3-6C)alkenyloxycarbonyl, cyano, nitro, (1-4C)alkanoyl, (1-4C)alkylthio, (1-4C)alkylsulphinyl, (1-4C)alkylsulphonyl or phenylsulphonyl; $R^5$ is hydrogen, (1-8C)alkyl, (1-4C)alkyl containing one or more fluoro substituents, hydroxy(1-4C)alkyl, carboxy, (1-4C)alkoxycarbonyl, (3-6C)alkenyloxycarbonyl, cyano, (1-4C)alkanoyl, (1-4C)alkylthio, (1-4C)alkylsulphinyl, (1-4C)alkylsulphonyl, phenylsulphonyl, (3-8C)cycloalkyl, (3-8C)cycloalkyl(1-4C)alkyl, phenyl or phenyl(1-4C)alkyl; $R^6$ is selected from hydrogen, (1-4C)alkyl, (1-4C)alkoxy, halogeno, trifluoromethyl, cyano and nitro; X is phenylene optionally bearing a substituent selected from (1-4C)alkyl, (1-4C)alkoxy, halogeno, (1-4C)alkanoyl, trifluoromethyl, cyano and nitro, or X is a direct bond between the adjacent phenyl and methylene groups; Z is 1H-tetrazol-5-yl, -CO.NH.(1H-tetrazol-5-yl) or a group of the formula **-CO.OR⁷** or **-CO.NH.SO₂.R⁸** in which $R^7$ is hydrogen or a non-toxic, biodegradable residue of a physiologically acceptable alcohol or phenol, and $R^8$ is (1-6C)alkyl, (3-8C)cycloalkyl or phenyl; and wherein any of said phenyl moieties may be unsubstituted or bear one or two substituents independently selected from (1-4C)alkyl, (1-4C)alkoxy, halogeno, cyano and trifluoromethyl; or an N-oxide thereof; or a non-toxic salt thereof; which is characterised by:-

(a) For those compounds of formula I in which Z is carboxy, a carboxylic acid derivative of the formula II

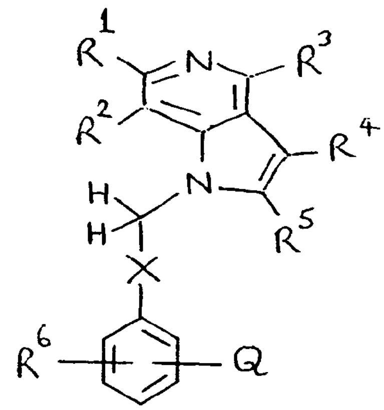

II

in which Q is a protected carboxy group selected from (1-6C)alkoxycarbonyl, phenoxycarbonyl, benzyloxycarbonyl and carbamoyl, is converted to carboxy;
(b) For those compounds of formula I wherein Z is tetrazolyl, a compound of formula III

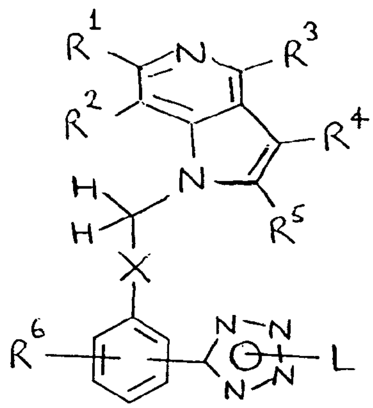

III

in which L is a suitable protecting group affixed to a nitrogen of the tetrazolyl moiety, is deprotected;
(c) A compound of the formula IV

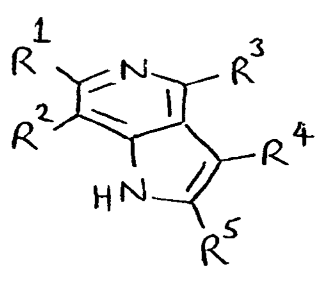

IV

is alkylated with a compound of the formula V

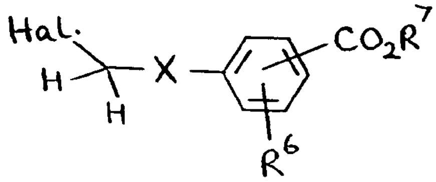

V

wherein **Hal.** stands for a suitable leaving group; or

(d) For a compound of the formula I wherein Z is tetrazolyl, X is p-phenylene optionally bearing a substituent selected from (1-4C)alkyl, (1-4C)alkoxy, halogeno, trifluoromethyl, cyano and nitro, a compound of the formula IX

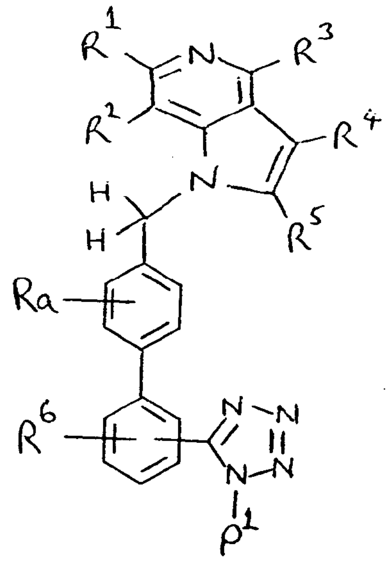

IX

wherein $P^1$ is an electron-deficient phenyl group; Ra is hydrogen, (1-4C)alkyl, (1-4C)alkoxy, halogeno, trifluoromethyl, cyano or nitro, is reacted with a base selected from an alkali metal hydroxide, (1-12C)alkanolate, (1-12C)alkanethiolate, phenolate, thiophenolate or diphenylphosphide, wherein any phenyl ring of the latter three groups may optionally bear a (1-4C)alkyl, (1-4C)alkoxy or halogeno group;

whereafter: when a compound of the formula I is required wherein Z is 1H-tetrazol-5-yl, a compound of the formula I wherein Z is a group of the formula -CO.OR$^7$ is converted into the corresponding nitrile under standard conditions, followed by reaction of the nitrile with an azide;

when a compound of the formula I is required wherein $R^3$ is hydrogen, a compound of the formula I wherein $R^3$ is halogeno is catalytically hydrogenated;

when a compound of the formula I is required wherein Z is a group of the formula **-CO.NH.SO$_2$R$^8$** or a group of the formula **-CO.OR$^7$** in which $R^7$ is other than hydrogen, a carboxylic acid of the formula I in which Z is carboxy (or a reactive derivative of said acid) is reacted with a sulphonamide of the formula **NH$_2$.SO$_2$R$^8$** or a hydroxy compound of the formula **HO.R$^7$**, or with a salt thereof;

when an N-oxide of a compound of the formula I is required, a compound of the formula I is oxidised;

when a non-toxic salt of a compound of formula I is required, it is obtained by reaction with the appropriate acid or base affording a physiologically acceptable ion, or by any other conventional salt formation procedure; and

when an optically active form of a compound of formula I is required, one of the aforesaid processes (a)-(d) is carried out using an optically active starting material, or the racemic form of a compound of formula I in which Z is an acidic group is resolved by reaction with an optically active form of a suitable organic base followed by conventional separation of the diastereoisomeric mixture of salts thus obtained, and liberation of the required optically active form of said compound of formula I by conventional treatment with acid;

and wherein $R^1$, $R^2$, $R^3$, $R^4$, $R^5$, $R^6$, $R^7$, $R^8$, Ra, X and Z have any of the meanings defined above unless otherwise stated.

**2.** A process as claimed in claim I wherein in the starting materials $R^1$ is hydrogen, methyl, ethyl, propyl, isopropyl, butyl, isobutyl, sec-butyl, pentyl, hexyl, cyclopropyl, cyclopentyl, cyclohexyl, phenyl, fluoromethyl, trifluoromethyl, 2,2,2-trifluoroethyl, pentafluoroethyl, cyclopropylmethyl, cyclopentylmethyl, cyclohexylmethyl, 2-methoxyethyl, 2-ethoxyethyl, benzyl, 1-phenylethyl or 2-phenylethyl; $R^2$ is methyl, ethyl, propyl, isopropyl, butyl, methoxy, ethoxy, fluoro, chloro, bromo, iodo, trifluoromethyl, carboxy, methoxycarbonyl, ethoxycarbonyl, propoxycarbonyl, allyloxycarbonyl, 2-methyl-2-propenyloxycarbonyl, 3-methyl-3-butenyloxycarbonyl, cyano, nitro, carbamoyl, formyl, acetyl, propionyl, N-methylcarbamoyl, N-ethylcarbamoyl, N,N-dimethylcarbamoyl, N,N-diethylcarbamoyl, amino, methylamino, ethylamino, butylamino, dimethylamino, diethylamino, dipropylamino, 3-methylureido, 3-ethylureido, 3-propylureido, formamido, acetamido or propanamido; $R^3$ is selected from hydrogen, methyl, ethyl, propyl, isopropyl, butyl, isobutyl, sec-butyl, pentyl, hexyl, cyclopropyl, cyclopentyl, cyclohexyl, phenyl, fluoromethyl, trifluoromethyl, 2,2,2-trifluoroethyl, pentafluoroethyl, cyclopropylmethyl, cyclopentylmethyl, cyclohexylmethyl, 2-methoxyethyl, 2-ethoxyethyl, benzyl, 1-phenylethyl, 2-phenylethyl, methoxy, ethoxy, fluoro, chloro, bromo, iodo, hydroxy, amino, methylamino, ethylamino, butylamino, dimethylamino and diethylamino; $R^4$ is hydrogen, methyl, ethyl, propyl, isopropyl, butyl, fluoromethyl, trifluoromethyl, 2,2,2-trifluoroethyl, pentafluoroethyl, methoxy, ethoxy, fluoro, chloro, bromo, iodo, carboxy, methoxycarbonyl, ethoxycarbonyl, propoxycarbonyl, allyloxycarbonyl, 2-methyl-2-propenyloxycarbonyl, 3-methyl-3-butenyloxycarbonyl, cyano, nitro, formyl, acetyl, propionyl, methythio, ethylthio, methylsulphinyl, ethylsulphinyl, methylsulphonyl, ethylsulphonyl or phenylsulphonyl; $R^5$ is hydrogen, methyl, ethyl, propyl, isopropyl, butyl, isobutyl, sec-butyl, pentyl, hexyl, fluoromethyl, trifluoromethyl, 2,2,2-trifluoroethyl, pentafluoroethyl, hydroxymethyl, 1-hydroxyethyl, 2-hydroxyethyl, carboxy, methoxycarbonyl, ethoxycarbonyl, propoxycarbonyl, allyloxycarbonyl, cyano, formyl, acetyl, propionyl, methythio, ethylthio, methylsulphinyl, ethylsulphinyl, methylsulphonyl, ethylsulphonyl or phenylsulphonyl, 2-methyl-2-propenyloxycarbonyl, 3-methyl-3-butenyloxycarbonyl, cyclopropyl, cyclopentyl, cyclohexyl, cyclopropylmethyl, cyclopentylmethyl, cyclohexylmethyl, 2-cyclopentylethyl, phenyl, benzyl, 1-phenylethyl or 2-phenylethyl; $R^6$ is hydrogen, methyl, ethyl, propyl, isopropyl, butyl, methoxy, ethoxy, fluoro, chloro, bromo, iodo, trifluoromethyl, cyano or nitro; X is phenylene optionally bearing a substituent selected from methyl, ethyl, methoxy, ethoxy, fluoro, chloro, bromo, iodo, formyl, acetyl, propionyl, trifluoromethyl, cyano or nitro, or X is a direct bond between the adjacent phenyl and methylene groups; $R^7$ is hydrogen or a residue derived from a (1-6C)alkanol, or phenol or glycerol; and $R^8$ is methyl, ethyl, propyl, isopropyl, butyl, pentyl, cyclobutyl, cyclopentyl, cyclohexyl or phenyl; and wherein any of said phenyl moieties may be unsubstituted or bear one or two substituents selected from methyl, ethyl, methoxy, ethoxy, fluoro, chloro, bromo, cyano and trifluoromethyl.

3. A process as claimed in claim 1 wherein in the starting materials $R^5$ is hydrogen, (1-8C)alkyl, hydroxy(1-4C)alkyl, carboxy, (1-4C)alkoxycarbonyl, (3-6C)alkenyloxycarbonyl, (3-8C)cycloalkyl, (3-8C)cycloalkyl(1-4C)alkyl, phenyl or phenyl(1-4C)alkyl, and wherein the phenyl rings of the latter two groups may optionally bear one or two substituents independently selected from (1-4C)alkyl, (1-4C)alkoxy, halogeno, cyano and trifluoromethyl.

4. A compound of the formula III wherein $R^1$, $R^2$, $R^3$, $R^4$, $R^5$ $R^6$ and X have any of the meanings defined in claim 1, and L is a protecting group.

5. The use of a compound of formula I, or a non-toxic salt thereof, as defined in claim 1, in the manufacture of a novel medicament.

**Claims for the following Contracting State : ES**

1. A process for the manufacture of a pyrrolopyridine derivative of the formula I

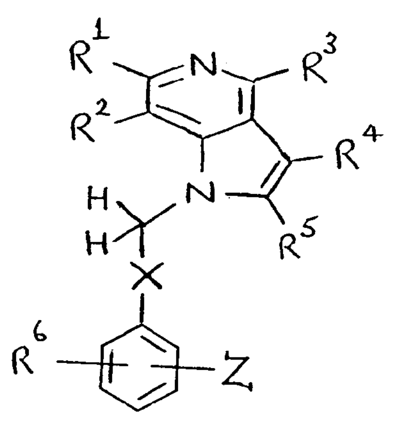

I

wherein $R^1$ is hydrogen, (1-8C)alkyl, (3-8C)cycloalkyl, phenyl or substituted (1-4C)alkyl, the latter containing one or more fluoro substituents or bearing a (3-8C)cycloalkyl, (1-4C)alkoxy or phenyl substituent; $R^2$

is hydrogen, (1-4C)alkyl, (1-4C)alkoxy, halogeno, trifluoromethyl, carboxy, (1-4C)alkoxycarbonyl, (3-6C)alkenyloxycarbonyl, cyano, nitro, carbamoyl, (1-4C)alkanoyl, N-alkylcarbamoyl and di-(N-alkyl)carbamoyl of up to 7 carbon atoms, amino, alkylamino and dialkylamino of up to 6 carbon atoms, 3-(1-4C)alkylureido and (1-4C)alkanoylamino; $R^3$ is selected from halogeno, (1-4C)alkoxy, hydroxy, amino, alkylamino and dialkylamino of up to 6 carbon atoms, and any of the values defined for $R^1$; $R^4$ is hydrogen, (1-4C)alkyl, (1-4C)alkyl containing one or more fluoro substituents, (1-4C)alkoxy, halogeno, carboxy, (1-4C)alkoxycarbonyl, (3-6C)alkenyloxycarbonyl, cyano, nitro, (1-4C)alkanoyl, (1-4C)alkylthio, (1-4C)alkylsulphinyl, (1-4C)alkylsulphonyl or phenylsulphonyl; $R^5$ is hydrogen, (1-8C)alkyl, (1-4C)alkyl containing one or more fluoro substituents, hydroxy(1-4C)alkyl, carboxy, (1-4C)alkoxycarbonyl, (3-6C)alkenyloxycarbonyl, cyano, (1-4C)alkanoyl, (1-4C)alkylthio, (1-4C)alkylsulphinyl, (1-4C)alkylsulphonyl, phenylsulphonyl, (3-8C)cycloalkyl, (3-8C)cycloalkyl(1-4C)alkyl, phenyl or phenyl(1-4C)alkyl; $R^6$ is selected from hydrogen, (1-4C)alkyl, (1-4C)alkoxy, halogeno, trifluoromethyl, cyano and nitro; X is phenylene optionally bearing a substituent selected from (1-4C)alkyl, (1-4C)alkoxy, halogeno, (1-4C)alkanoyl, trifluoromethyl, cyano and nitro, or X is a direct bond between the adjacent phenyl and methylene groups; Z is 1H-tetrazol-5-yl, -CO.NH.(1H-tetrazol-5-yl) or a group of the formula **-CO.OR⁷** or **-CO.NH.SO₂.R⁸** in which $R^7$ is hydrogen or a non-toxic, biodegradable residue of a physiologically acceptable alcohol or phenol, and $R^8$ is (1-6C)alkyl, (3-8C)cycloalkyl or phenyl; and wherein any of said phenyl moieties may be unsubstituted or bear one or two substituents independently selected from (1-4C)alkyl, (1-4C)alkoxy, halogeno, cyano and trifluoromethyl; or an N-oxide thereof; or a non-toxic salt thereof; which is characterised by:-

(a) For those compounds of formula I in which Z is carboxy, a carboxylic acid derivative of the formula II

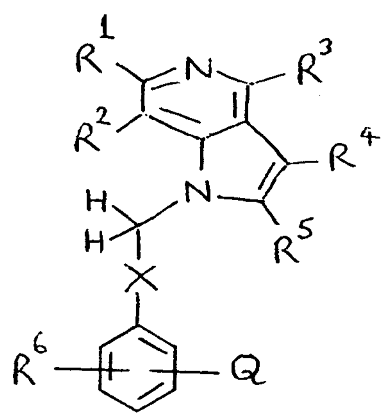

II

in which Q is a protected carboxy group selected from (1-6C)alkoxycarbonyl, phenoxycarbonyl, benzyloxycarbonyl and carbamoyl, is converted to carboxy;

(b) For those compounds of formula I wherein Z is tetrazolyl, a compound of formula III

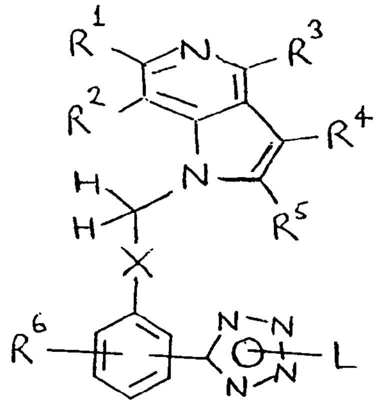

III

in which L is a suitable protecting group affixed to a nitrogen of the tetrazolyl moiety, is deprotected;

(c) A compound of the formula IV

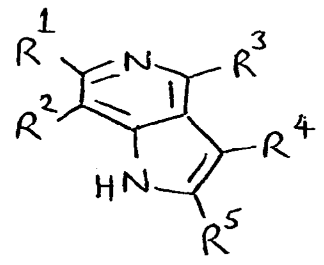

IV

is alkylated with a compound of the formula V

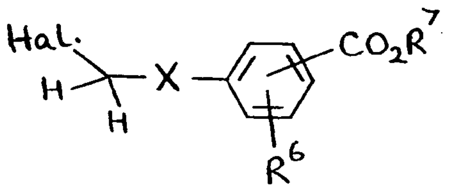

V

wherein **Hal.** stands for a suitable leaving group; or

(d) For a compound of the formula I wherein Z is tetrazolyl, X is p-phenylene optionally bearing a substituent selected from (1-4C)alkyl, (1-4C)alkoxy, halogeno, trifluoromethyl, cyano and nitro, a compound of the formula IX

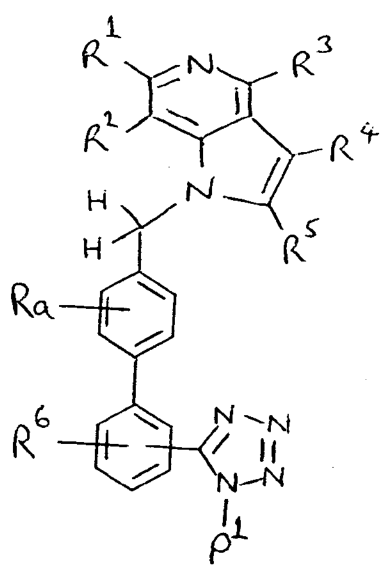

IX

wherein $P^1$ is an electron-deficient phenyl group; Ra is hydrogen, (1-4C)alkyl, (1-4C)alkoxy, halogeno, trifluoromethyl, cyano or nitro, is reacted with a base selected from an alkali metal hydroxide, (1-12C)alkanolate, (1-12C)alkanethiolate, phenolate, thiophenolate or diphenylphosphide, wherein any phenyl ring of the latter three groups may optionally bear a (1-4C)alkyl, (1-4C)alkoxy or halogeno group;

whereafter: when a compound of the formula I is required wherein Z is 1H-tetrazol-5-yl, a compound of the formula I wherein Z is a group of the formula $-CO.OR^7$ is converted into the corresponding nitrile under standard conditions, followed by reaction of the nitrile with an azide;

when a compound of the formula I is required wherein $R^3$ is hydrogen, a compound of the formula I wherein $R^3$ is halogeno is catalytically hydrogenated;

when a compound of the formula I is required wherein Z is a group of the formula **$-CO.NH.SO_2R^8$** or a group of the formula **$-CO.OR^7$** in which $R^7$ is other than hydrogen, a carboxylic acid of the formula I in which Z is carboxy (or a reactive derivative of said acid) is reacted with a sulphonamide of the formula **$NH_2.SO_2R^8$** or a hydroxy compound of the formula **$HO.R^7$**, or with a salt thereof;

when an N-oxide of a compound of the formula I is required, a compound of the formula I is oxidised;

when a non-toxic salt of a compound of formula I is required, it is obtained by reaction with the appropriate acid or base affording a physiologically acceptable ion, or by any other conventional salt formation procedure; and

when an optically active form of a compound of formula I is required, one of the aforesaid processes (a)-(d) is carried out using an optically active starting material, or the racemic form of a compound of formula I in which Z is an acidic group is resolved by reaction with an optically active form of a suitable organic base followed by conventional separation of the diastereoisomeric mixture of salts thus obtained, and liberation of the required optically active form of said compound of formula I by conventional treatment with acid;

and wherein $R^1$, $R^2$, $R^3$, $R^4$, $R^5$, $R^6$, $R^7$, $R^8$, Ra, X and Z have any of the meanings defined above unless otherwise stated.

**2.** A process as claimed in claim 1 wherein in the starting materials $R^1$is hydrogen, methyl, ethyl, propyl, isopropyl, butyl, isobutyl, sec-butyl, pentyl, hexyl, cyclopropyl, cyclopentyl, cyclohexyl, phenyl, fluoromethyl, trifluoromethyl, 2,2,2-trifluoroethyl, pentafluoroethyl, cyclopropylmethyl, cyclopentylmethyl, cyclohexylmethyl, 2-methoxyethyl, 2-ethoxyethyl, benzyl, 1-phenylethyl or 2-phenylethyl; $R^2$ is methyl, ethyl, propyl, isopropyl, butyl, methoxy, ethoxy, fluoro, chloro, bromo, iodo, trifluoromethyl, carboxy, methoxycarbonyl, ethoxycarbonyl, propoxycarbonyl, allyloxycarbonyl, 2-methyl-2-propenyloxycarbonyl, 3-methyl-3-butenyloxycarbonyl, cyano, nitro, carbamoyl, formyl, acetyl, propionyl, N-methylcarbamoyl, N-ethylcarbamoyl, N,N-dimethylcarbamoyl, N,N-diethylcarbamoyl, amino, methylamino, ethylamino, butylamino, dimethylamino, diethylamino, dipropylamino, 3-methylureido, 3-ethylureido, 3-propylureido, formamido, acetamido or propanamido; $R^3$ is selected from hydrogen, methyl, ethyl, propyl, isopropyl, butyl, isobutyl, sec-butyl, pentyl, hexyl, cyclopropyl, cyclopentyl, cyclohexyl, phenyl, fluoromethyl, trifluoromethyl, 2,2,2-trifluoroethyl, pentafluoroethyl, cyclopropylmethyl, cyclopentylmethyl, cyclohexylmethyl, 2-methoxyethyl, 2-ethoxyethyl, benzyl, 1-phenylethyl, 2-phenylethyl, methoxy, ethoxy, fluoro, chloro, bromo, iodo, hydroxy, amino, methylamino, ethylamino, butylamino, dimethylamino and diethylamino; $R^4$ is hydrogen, methyl, ethyl, propyl, isopropyl, butyl, fluoromethyl, trifluoromethyl, 2,2,2-trifluoroethyl, pentafluoroethyl, methoxy, ethoxy, fluoro, chloro, bromo, iodo, carboxy, methoxycarbonyl, ethoxycarbonyl, propoxycarbonyl, allyloxycarbonyl, 2-methyl-2-propenyloxycarbonyl, 3-methyl-3-butenyloxycarbonyl, cyano, nitro, formyl, acetyl, propionyl, methythio, ethylthio, methylsulphinyl, ethylsulphinyl, methylsulphonyl, ethylsulphonyl or phenylsulphonyl; $R^5$ is hydrogen, methyl, ethyl, propyl, isopropyl, butyl, isobutyl, sec-butyl, pentyl, hexyl, fluoromethyl, trifluoromethyl, 2,2,,2-trifluoroethyl, pentafluoroethyl, hydroxymethyl, 1-hydroxyethyl, 2-hydroxyethyl, carboxy, methoxycarbonyl, ethoxycarbonyl, propoxycarbonyl, allyloxycarbonyl, cyano, formyl, acetyl, propionyl, methythio, ethylthio, methylsulphinyl, ethylsulphinyl, methylsulphonyl, ethylsulphonyl or phenylsulphonyl, 2-methyl-2-propenyloxycarbonyl, 3-methyl-3-butenyloxycarbonyl, cyclopropyl, cyclopentyl, cyclohexyl, cyclopropylmethyl, cyclopentylmethyl, cyclohexylmethyl, 2-cyclopentylethyl, phenyl, benzyl, 1-phenylethyl or 2-phenylethyl; $R^6$ is hydrogen, methyl, ethyl, propyl, isopropyl, butyl, methoxy, ethoxy, fluoro, chloro, bromo, iodo, trifluoromethyl, cyano or nitro; X is phenylene optionally bearing a substituent selected from methyl, ethyl, methoxy, ethoxy, fluoro, chloro, bromo, iodo, formyl, acetyl, propionyl, trifluoromethyl, cyano or nitro, or X is a direct bond between the adjacent phenyl and methylene groups; $R^7$ is hydrogen or a residue derived from a (1-6C)alkanol, or phenol or glycerol; and $R^8$ is methyl, ethyl, propyl, isopropyl, butyl, pentyl, cyclobutyl, cyclopentyl, cyclohexyl or phenyl; and wherein any of said phenyl moieties may be unsubstituted or bear one or two substituents selected from methyl, ethyl, methoxy, ethoxy, fluoro, chloro, bromo, cyano and trifluoromethyl.

**3.** A process as claimed in claim 1 wherein in the starting materials $R^5$ is hydrogen, (1-8C)alkyl, hydroxy(1-4C)alkyl, carboxy, (1-4C)alkoxycarbonyl, (3-6C)alkenyloxycarbonyl, (3-8C)cycloalkyl, (3-8C)cycloalkyl(1-4C)alkyl, phenyl or phenyl(1-4C)alkyl, and wherein the phenyl rings of the latter two groups may optionally bear one or two substituents independently selected from (1-4C)alkyl, (1-4C)alkoxy, halogeno, cyano and trifluoromethyl.

**4.** The use of a compound of formula I, or a non-toxic salt thereof, as defined in claim 1, in the manufacture of a novel medicament.